# EUROPEAN PATENT APPLICATION

(11) **EP 3 167 896 A1**
(43) Date of publication of application: **17.05.2017**
(21) Application number: 16190808.2
(22) Date of filing: 25.02.2011
(51) Int. Cl.: A61K 38/06, A61P 39/06, A61P 21/00

(54) **MITOCHONDRIAL-TARGETED ANTIOXIDANTS PROTECT AGAINST MECHANICAL VENTILATION-INDUCED DIAPHRAGM DYSFUNCTION AND SKELETAL MUSCLE ATROPHY**

(30) Priority: 26.02.2010 US 308508 P
(62) Divisional of application: 11748191.1
(71) Applicant: University of Florida Research Foundation, Inc., Gainesville, FL 32611 (US); Cornell University, Ithaca, NY 14850 (US)
(72) Inventor: SZETO, Hazel, New York, NY New York 10128 (US); POWERS, Scott Kline, Gainesville, FL Florida 32608 (US)
(74) Representative: Atkinson, Jennifer

(57) **Abstract**

The present disclosure provides methods and compositions for preventing or treating MV-induced or disuse-induced skeletal muscle infirmities in a mammalian subject. The methods further include administering to the subject an effective amount of an aromatic-cationic peptide.

## Description

### GOVERNMENT SUPPORT

This invention was made with government support under grant R01HL08783 awarded by the National Institute of Health. The government has certain rights in the invention.

### CROSS-REFERENCE TO RELATED APPLICATIONS

This application claims priority to U.S. Provisional Application No. 61/308,508, filed February 26, 2010, which is incorporated herein by reference in its entirety.

### TECHNICAL FIELD

Disclosed herein are methods and compositions that include aromatic-cationic peptides useful for the prevention and treatment of skeletal muscle infirmities, such as weakness, dysfunction and/or muscle atrophy. In particular, methods and compositions for the prevention and treatment of mechanical ventilation (MV)-induced diaphragm infirmities, and disuse-induced skeletal muscle infirmities are disclosed.

### BACKGROUND

The following description is provided to assist the understanding of the reader. None of the information provided or references cited is admitted to be prior art to the present invention.

Mechanical ventilation (MV) is clinically employed to achieve adequate pulmonary gas exchange in subjects incapable of maintaining sufficient alveolar ventilation. Common indications for MV include respiratory failure, heart failure, surgery, drug overdose, and spinal cord injuries. Even though MV is a life-saving measure for subjects with respiratory failure, complications associated with weaning patients from MV are common. Indeed, weaning difficulties are an important clinical problem; 20-30% of mechanically ventilated subjects experience weaning difficulties. The "failure to wean" may be due to several factors including respiratory muscle weakness of the diaphragm, a skeletal muscle.

Skeletal muscle weakness emanate from muscle fiber atrophy and dysfunction. In this regard, muscle disuse presents a widespread problem for individuals subject to body or limb immobilization, *e.g.*, muscle constraints due to bone fracture casting or prolonged MV. Such muscle disuse, however, does not elucidate the etiology of muscle fiber degradation at the cellular level. To this end, oxidative stress, such as the generation of reactive oxygen species (ROS) *via* xanthine oxidase activation, may impart a mechanism for skeletal muscle degradation and contractile dysfunction. However, inhibition of xanthine oxidase activity does not completely protect against the effects of skeletal muscle disuse-induced or MV-induced oxidative stress, concomitant atrophy and weakness. Accordingly, identifying additional factors associated with muscle dysfunction and atrophy are considerations in the development of new strategies for preventing or treating these ailments.

### SUMMARY

Disclosed herein are methods and compositions for the prevention and treatment of skeletal muscle infirmities, such as mechanical ventilation (MV)-induced diaphragm weakness, dysfunction and/or atrophy. Generally, the methods and compositions include one or more aromatic-cationic peptides or a pharmaceutically acceptable salt there of, *(e.g.,* acetate or trifluoroacetate salt), and in some embodiments, a therapeutically effective amount of one or more aromatic-cationic peptides or a pharmaceutically acceptable salt thereof, *(e.g.,* acetate or trifluoroacetate salt) is administered to a subject in need thereof, to treat or prevent or treat skeletal muscle infirmity such as weakness, dysfunction and/or atrophy.

Disclosed herein are methods and compositions for the prevention and treatment of skeletal muscle infirmities, such as mechanical ventilation (MV)-induced diaphragm weakness, dysfunction and/or atrophy, and/or disuse induced muscle infirmities. Generally, the methods and compositions include one or more aromatic-cationic peptides or a pharmaceutically acceptable salt thereof, (*e.g*., acetate or trifluoroacetate salt), and in some embodiments, a therapeutically effective amount of one or more aromatic-cationic peptides or a pharmaceutically acceptable salt there of, *(e.g.,* acetate or trifluoroacetate salt) is administered to a subject in need thereof, to treat or prevent skeletal muscle infirmities.

In some aspects, methods for treating or preventing skeletal muscle infirmities in a mammalian subject are provided. Typically, the methods include administering to the mammalian subject a therapeutically effective amount of the peptide D-Arg-2',6'Dmt-Lys-Phe-NH₂, or a pharmaceutically acceptable salt thereof, (*e.g.*, acetate or trifluoroacetate salt). In some embodiments, the peptide is administered orally, topically, systemically, intravenously, subcutaneously, intraperitoneally, or intramuscularly.

In some embodiments, the skeletal muscle comprises diaphragmatic muscle, and the skeletal muscle infirmity results from mechanical ventilation (MV). In some embodiments, a method of treating or preventing MV-induced diaphragm dysfunction in a mammalian subject is provided. In some embodiments, the duration of the MV is at least 10 hours, and in some embodiments, the peptide is administered to the subject prior to MV, during the MV, or both prior to and during the MV. In some embodiments, the peptide is administered orally, topically, systemically, intravenously, subcutaneously, intraperitoneally, or intramuscularly

Additionally or alternatively, in some embodiments, methods of treating or preventing disuse-induced skeletal muscle atrophy in a mammalian subject are provided. Typically, such methods include administering to the mammalian subject a therapeutically effective amount of the peptide D-Arg-2',6'Dmt-Lys-Phe-NH₂ or a pharmaceutically acceptable salt thereof (e.g., acetate or trifluoroacetate salt). In some embodiments, the skeletal muscle includes soleus muscle or plantaris muscle, or both the soleus and plantaris muscle. In some embodiments, the peptide is administered to the subject prior to or during the disuse. In some embodiments, the peptide is administered orally, topically, systemically, intravenously, subcutaneously, intraperitoneally, or intramuscularly

Additionally or alternatively, in some embodiments, methods for treating a disease or condition characterized by increased oxidative damage in skeletal muscle of a mammalian subject are provided. Typically, such methods include administering to the subject an effective amount of D-Arg-2',6'Dmt-Lys-Phe-NH₂ or a pharmaceutically acceptable salt thereof (e.g., acetate or trifluoroacetate salt). In some embodiments, the peptide is administered to the subject prior to or during the increased oxidative damage. In some embodiments, the oxidative damage is associated with a variation in the gene expression or protein levels, activity, or degradation of one or more biomarkers compared to a control level. In some embodiments, the control level is the levels of the one or more biomarkers from a healthy individual not afflicted with disuse-induced skeletal muscle atrophy or MV-induced diaphragm dysfunction. In some embodiments, the biomarkers are selected from the group consisting of calpain, caspase-3, caspase-12, 20S proteasome, E3 ligases, atrogin-1/MAFbx, MuRF-1, αII-spectrin, sarcomeric protein, 4-HNE-conjugated cytosolic proteins, and protein carbonyls in myofibrillar proteins. In some embodiments, the disease or condition characterized by increased oxidative damage includes disuse-induced skeletal muscle atrophy or MV-induced diaphragm dysfunction. In some embodiments, the peptide is administered orally, topically, systemically, intravenously, subcutaneously, intraperitoneally, or intramuscularly

In one aspect, the disclosure provides a method of treating or preventing MV-induced diaphragm dysfunction, comprising administering to a mammalian subject in need thereof a therapeutically effective amount of an aromatic-cationic peptide. In some embodiments, the aromatic-cationic peptide is a peptide including:
at least one net positive charge;
a minimum of four amino acids;
a maximum of about twenty amino acids;
a relationship between the minimum number of net positive charges (pm) and the total number of amino acid residues (r) wherein 3pₘ is the largest number that is less than or equal to r + 1; and a relationship between the minimum number of aromatic groups (a) and the total number of net positive charges (pt) wherein 2a is the largest number that is less than or equal to pt + 1, except that when a is 1, pt may also be 1. In some embodiments, the mammalian subject is a human.

In one embodiment, 2pₘ is the largest number that is less than or equal to r+1, and a may be equal to pt. The aromatic-cationic peptide may be a water-soluble peptide having a minimum of two or a minimum of three positive charges.

In one embodiment, the peptide comprises one or more non-naturally occurring amino acids, for example, one or more D-amino acids. In some embodiments, the C-terminal carboxyl group of the amino acid at the C-terminus is amidated. In certain embodiments, the peptide has a minimum of four amino acids. The peptide may have a maximum of about 6, a maximum of about 9, or a maximum of about 12 amino acids.

In one embodiment, the peptide comprises a tyrosine or a 2',6'-dimethyltyrosine (Dmt) residue at the N-terminus. For example, the peptide may have the formula Tyr-D-Arg-Phe-Lys-NH2 (SS-01) or 2',6'-Dmt-D-Arg-Phe-Lys-NH2 (SS-02). In another embodiment, the peptide comprises a phenylalanine or a 2',6'-dimethylphenylalanine residue at the N-terminus. For example, the peptide may have the formula Phe-D-Arg-Phe-Lys-NH2 (SS-20) or 2',6'-Dmp-D-Arg-Phe-Lys-NH2. In a particular embodiment, the aromatic-cationic peptide has the formula D-Arg-2',6'-Dmt-Lys-Phe-NH₂ (SS-31).

In one embodiment, the peptide is defined by formula I. 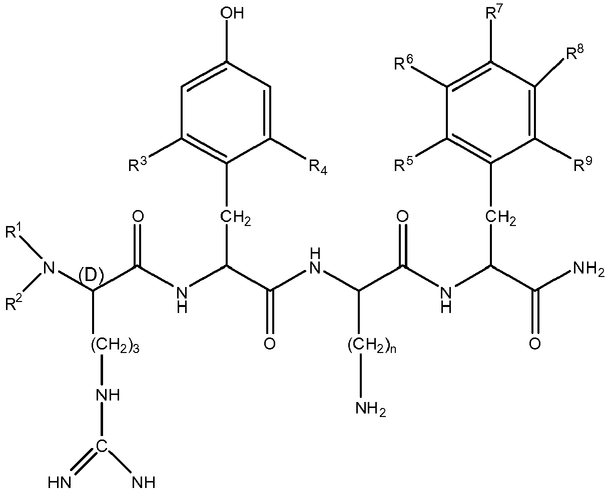 wherein R¹ and R² are each independently selected from
(i) hydrogen;
(ii) linear or branched C₁-C₆ alkyl;
(iii) 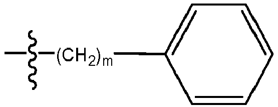 where m = 1-3;
(iv) 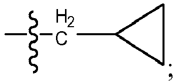
(v) 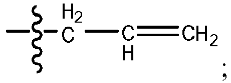
R³ and R⁴ are each independently selected from
(i) hydrogen;
(ii) linear or branched C₁-C₆ alkyl;
(iii) C₁-C₆ alkoxy;
(iv) amino;
(v) C₁-C₄ alkylamino;
(vi) C₁-C₄ dialkylamino;
(vii) nitro;
(viii) hydroxyl;
(ix) halogen, where "halogen" encompasses chloro, fluoro, bromo, and iodo;
R⁵, R⁶, R⁷, R⁸, and R⁹ are each independently selected from
(i) hydrogen;
(ii) linear or branched C₁-C₆ alkyl;
(iii) C₁-C₆ alkoxy;
(iv) amino;
(v) C₁-C₄ alkylamino;
(vi) C₁-C₄ dialkylamino;
(vii) nitro;
(viii) hydroxyl;
(ix) halogen, where "halogen" encompasses chloro, fluoro, bromo, and iodo; and n is an integer from 1 to 5.

In a particular embodiment, R¹ and R² are hydrogen; R³ and R⁴ are methyl; R⁵, R⁶, R⁷, R⁸, and R⁹ are all hydrogen; and n is 4.

In one embodiment, the peptide is defined by formula II: 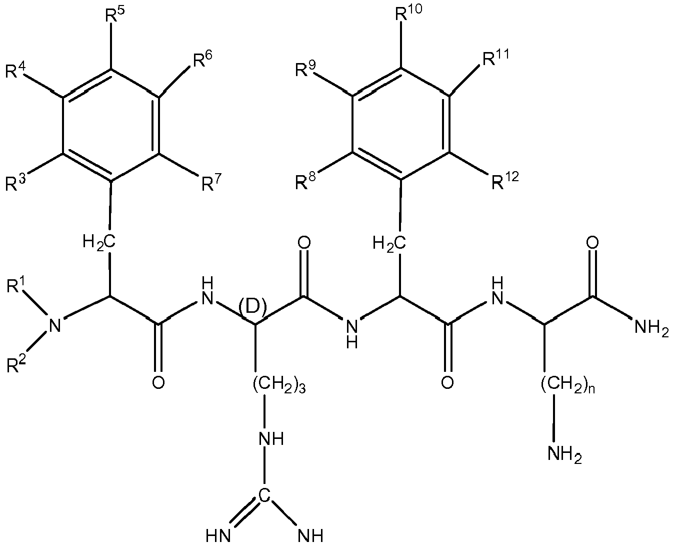 wherein R¹ and R² are each independently selected from
(i) hydrogen;
(ii) linear or branched C₁-C₆ alkyl;
(iii) 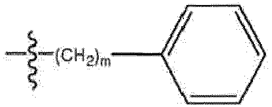 where m = 1-3;
(iv) 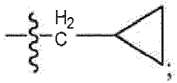
(v) 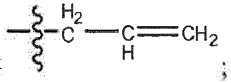
R³, R⁴, R⁵, R⁶, R⁷, R⁸, R⁹, R¹⁰, R¹¹ and R¹² are each independently selected from
(i) hydrogen;
(ii) linear or branched C₁-C₆ alkyl;
(iii) C₁-C₆ alkoxy;
(iv) amino;
(v) C₁-C₄ alkylamino;
(vi) C₁-C₄ dialkylamino;
(vii) nitro;
(viii) hydroxyl;
(ix) halogen, where "halogen" encompasses chloro, fluoro, bromo, and iodo; and n is an integer from 1 to 5.

In a particular embodiment R¹, R², R³, R⁴, R⁵, R⁶, R⁷, R⁸, R⁹, R¹⁰, R¹¹, and R¹² are all hydrogen; and n is 4. In another embodiment, R¹, R², R³, R⁴, R⁵, R⁶, R⁷, R⁸, R⁹, and R¹¹ are all hydrogen; R⁸ and R¹² are methyl; R¹⁰ is hydroxyl; and n is 4.

The aromatic-cationic peptides may be administered in a variety of ways. In some embodiments, the peptides are administered orally, topically, intranasally, intraperitoneally, intravenously, or subcutaneously.

The application provides amongst other things the following clauses:
1. A method of treating or preventing skeletal muscle infirmities in a mammalian subject, comprising administering to the mammalian subject a therapeutically effective amount of the peptide D-Arg-2',6'Dmt-Lys-Phe-NH₂ or a pharmaceutically acceptable salt thereof.
2. The method of clause 1, wherein the skeletal muscle comprises diaphragmatic muscle.
3. The method of clause 1, wherein the skeletal muscle infirmity results from mechanical ventilation (MV).
4. The method of clause 3, wherein the duration of the MV is at least 10 hours.
5. The method of clause 3, wherein the peptide is administered to the subject prior to MV, during the MV or both.
6. The method of clause 1, wherein the peptide is administered orally, topically, systemically, intravenously, subcutaneously, intraperitoneally, or intramuscularly.
7. A method of treating or preventing MV-induced diaphragm dysfunction in a mammalian subject, comprising administering to the mammalian subject a therapeutically effective amount of the peptide D-Arg-2',6'Dmt-Lys-Phe-NH₂ or a pharmaceutically acceptable salt thereof.
8. The method of clause 7, wherein the peptide is administered to the subject prior to MV, during MV, or both.
9. The method of clause 7, wherein the MV is at least 10 hours.
10. The method of clause 7, wherein the peptide is administered orally, topically, systemically, intravenously, subcutaneously, intraperitoneally, or intramuscularly.
11. A method of treating or preventing disuse-induced skeletal muscle atrophy in a mammalian subject, comprising administering to the mammalian subject a therapeutically effective amount of the peptide D-Arg-2',6'Dmt-Lys-Phe-NH₂ or a pharmaceutically acceptable salt thereof.
12. The method of clause 11, wherein the skeletal muscle comprises soleus muscle or plantaris muscle, or both soleus and plantaris muscle.
13. The method of clause 11, wherein the peptide is administered to the subject prior to or during the disuse.
14. The method of clause 11, wherein the peptide is administered orally, topically, systemically, intravenously, subcutaneously, intraperitoneally, or intramuscularly.
15. A method for treating a disease or condition characterized by increased oxidative damage in skeletal muscle of a mammalian subject in need thereof, the method comprising:
   administering to the subject an effective amount of D-Arg-2',6'Dmt-Lys-Phe-NH₂ or a pharmaceutically acceptable salt thereof, wherein the oxidative damage is associated with a variation in the gene expression or protein levels, activity, or degradation of one or more biomarkers selected from the group consisting of calpain, caspase-3, caspase 12, 20S proteasome, E3 li gases, atrogin-1/MAFbx, MuRF-1, αII-spectrin, sarcomeric protein, 4-FiNE-conjugated cytosolic proteins, and protein carbonyls in myofibrillar proteins, compared to a control level.
16. The method of clause 15, wherein the disease or condition characterized by increased oxidative damage comprises disuse-induced skeletal muscle atrophy or MV-induced diaphragm dysfunction.
17. The method of clause 15, wherein the control level is the levels of the one or more biomarkers from a healthy individual not afflicted with disuse-induced skeletal muscle atrophy or MV-induced diaphragm dysfunction.
18. The method of clause 15, wherein the peptide is administered to the subject prior to or during the increased oxidative damage.
19. The method of clause 15, wherein the peptide is administered orally, topically, systemically, intravenously, subcutaneously, intraperitoneally, or intramuscularly.

### BRIEF DESCRIPTION OF THE DRAWINGS

FIG.1A and 1B are graphs illustrating the rates of hydrogen peroxide release from mitochondria isolated from diaphragms of control, mechanically ventilated (MV), and mechanically ventilated rats treated with the mitochondrial-targeted antioxidant SS-31
   (MVSS). FIG. 1A shows state 3 mitochondrial respiration. FIG. 1B shows state 4 mitochondrial respiration.

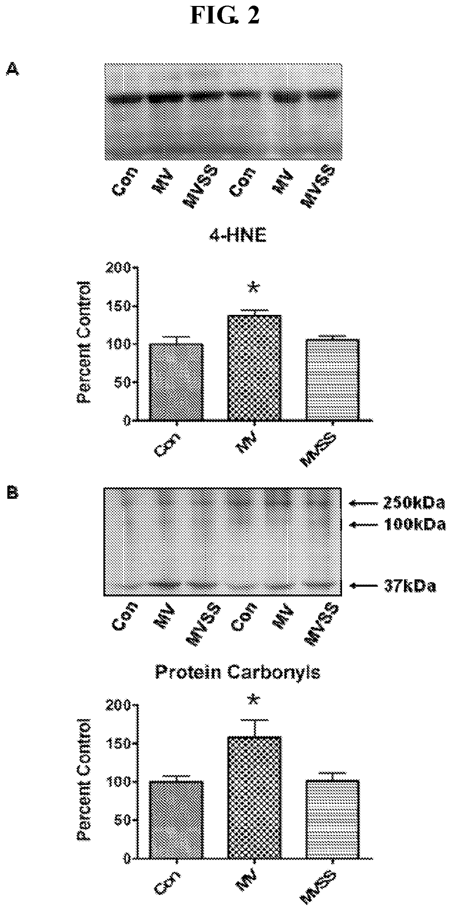

FIG. 2A and 2B are graphs showing the levels of oxidatively modified proteins in the diaphragm of control, MV, and mechanically ventilated rats treated with the mitochondrial-targeted antioxidant SS-31 (MVSS). FIG. 2A shows the levels of 4-hydroxyl-nonenal-conjugated proteins in the diaphragm of the three experimental groups. The image above the histograph is a representative western blot of data from the three experimental groups. FIG. 2B shows the levels of protein carbonyls in the diaphragm of the three experimental groups. The image above the histograph is a representative western blot of data from the three experimental groups.
FIG. 3 is a graph demonstrating the effects of prolonged MV on the diaphragmatic force-frequency response (*in vitro*) in control and mechanically ventilated rats in the presence and absence of mitochondrial targeted antioxidants.
FIG. 4 is a graph showing the fiber cross-sectional area (CSA) in diaphragm muscle myofibers from control and mechanically ventilated rats with (MVSS).
FIG. 5A- 5C are graphs showing protease activity. FIG. 5A shows the activity of the 20S proteasome. FIG. 5B shows the mRNA and protein levels of atrogin-1. FIG. 5C shows the mRNA and protein levels of MuRF-1. The images above the histograms in FIGS 5B and 5C are representative western blots of data from the three experimental groups.
FIG. 6A and 6B are graphs of calpain 1 and caspase 3 activity in the diaphragm from control and mechanically ventilated animals in the presence and absence of mitochondrial-targeted antioxidants (MVSS). FIG. 6A shows the active form of calpain 1 in diaphragm muscle at the completion of 12 hours of MV. FIG. 5B shows the cleaved and active band of caspase-3 in diaphragm muscle at the completion of 12 hours of MV. The images above the histograms are representative western blots of data from the three experimental groups.
FIG. 7A and 7B are graphs illustrating calpain and caspase-3 activity in the diaphragm from control and mechanically ventilated animals in the presence and absence of a mitochondrial-targeted antioxidants (MV). FIG. 7A shows levels of the 145 kDa α-II-spectrin break-down product (SBPD) in diaphragm muscle following 12 hours of MV. FIG. 7B shows the levels of the 120 kDa α-II-spectrin break-down product (SBPD 120 kDa) in diaphragm muscle following 12 hours of MV. The images above the histograms are representative western blots of data from the three experimental groups.
FIG. 8 is a graph showing the ratio of actin to total sarcomeric protein levels in the diaphragm from control and mechanically ventilated animals in the presence and absence of mitochondrial-targeted antioxidants (MV). The image above the histogram is a representative western blot of data from the three experimental groups.
FIG. 9A-9D are graphs showing that a mitochondrial-targeted antioxidant (SS-31) had no effect on soleus muscle weight (FIG. 9A), respiratory control ratio or RCR (FIG. 9B), mitochondrial state 3 respiration (FIG. 9C) or mitochondrial state 4 respiration (FIG. 9D) in normal muscle.
FIG. 10A-10C are graphs showing that a mitochondrial-targeted antioxidant (SS-31) had no effect on soleus muscle Type I (FIG. 10A), Type IIa (FIG. 10B), or Type IIb/x (FIG. 10C) fiber size (cross sectional area) in normal soleus muscle.
FIG. 11A-11D are graphs showing that a mitochondrial-targeted antioxidant (SS-31) had no effect on plantaris muscle weight (FIG. 11A), respiratory control ratio or RCR (FIG. 11B), mitochondrial state 3 respiration (FIG. 11C) or mitochondrial state 4 respiration (FIG. 11D) in normal muscle.
FIG. 12A and 12B are graphs showing that a mitochondrial-targeted antioxidant (SS-31) had no effect on plantaris muscle Type IIa (FIG. 12A) or Type IIb/x (FIG. 12B) fiber size (cross sectional area) in normal plantaris muscle.
FIG. 13A-13D are graphs illustrating that casting for 7 days caused significant decrease in weight of soleus muscle (FIG. 13A) which was prevented by SS-31. Casting also significantly reduced mitochondrial state 3 (FIG. 13C) respiration, but had no effect on state 4 (FIG. 13D), thus resulting in a significant decrease in RCR (FIG. 13B). All of the foregoing defects were prevented by SS-31.
FIG. 14A and 14B are graphs showing that casting for 7 days significantly increased H₂O₂ production by mitochondrial isolated from soleus muscle, which was prevented by SS-31 (FIG. 14A). FIG. 14B illustrates that SS-31 prevented the loss of cross sectional area of all three types of fibers as shown.
FIG. 15A-15D are graphs showing that casting for 7 days increased oxidative damage in soleus muscle, as measured by lipid peroxidation (FIG. 15A), which was blocked by SS-31. Casting also significantly increased protease activity of calpain-1 (FIG. 15B), caspase-3 (FIG. 15C) and caspase-12 (FIG. 15D) in the soleus muscle, which was prevented by SS-31.
FIG. 16A-16D are graphs showing that casting for 7 days reduced plantaris weight (FIG. 16A) and mitochondrial RCR (FIG. 16B) in the plantaris muscle, which was prevented by SS-31. FIG. 16C shows state 3 respiration, and FIG. 16D shows state 4 respiration.
FIG. 17 is a graph showing that casting for 7 days significantly increased H₂O₂ production by mitochondrial isolated from plantaris muscle, which was prevented by SS-31 (FIG. 17A). FIG. 17B illustrates that SS-31 prevented the loss of cross sectional area of two types of fibers as shown.
FIG. 18A-18D are graphs showing that casting for 7 days increased oxidative damage in plantaris muscle, as measured by lipid peroxidation (FIG. 18A), which was blocked by SS-31. Casting also increased protease activity of calpain-1 (FIG. 18B), caspase-3 (FIG. 18C) and caspase-12 (FIG. 18D) in the plantaris muscle, which was prevented by SS-31.

### DETAILED DESCRIPTION

It is to be appreciated that certain aspects, modes, embodiments, variations and features of the invention are described below in various levels of detail in order to provide a substantial understanding of the present invention. The definitions of certain terms as used in this specification are provided below. Unless defined otherwise, all technical and scientific terms used herein generally have the same meaning as commonly understood by one of ordinary skill in the art to which this invention belongs.

In practicing the present technology, many conventional techniques in molecular biology, protein biochemistry, cell biology, immunology, microbiology and recombinant DNA are used. These techniques are well-known and are explained in, *e.g.,* Current Protocols in Molecular Biology, Vols. I-III, Ausubel, Ed. (1997); Sambrook et al., Molecular Cloning: A Laboratory Manual, Second Ed. (Cold Spring Harbor Laboratory Press, Cold Spring Harbor, NY, 1989). All references cited herein are incorporated herein by reference in their entireties.

As used in this specification and the appended claims, the singular forms "a," "an" and "the" include plural referents unless the content clearly dictates otherwise. For example, reference to "a peptide" includes a combination of two or more peptides, and the like.

As used herein, phrases such as element A is "associated with" element B mean both elements exist, but should not be interpreted as meaning one element necessarily is causally linked to the other.

As used herein, the "administration" of an agent, drug, or peptide to a subject includes any route of introducing or delivering to a subject a compound to perform its intended function. Administration can be carried out by any suitable route, including orally, intranasally, parenterally (intravenously, intramuscularly, intraperitoneally, or subcutaneously), or topically. Administration includes self-administration and the administration by another.

As used herein, the term "amino acid" includes naturally-occurring amino acids, L-amino acids, D-amino acids, and synthetic amino acids, as well as amino acid analogs and amino acid mimetics that function in a manner similar to the naturally-occurring amino acids. Naturally-occurring amino acids are those encoded by the genetic code, as well as those amino acids that are later modified, *e.g*., hydroxyproline, γ-carboxyglutamate, and O-phosphoserine. Amino acid analogs refers to compounds that have the same basic chemical structure as a naturally-occurring amino acid, *e.g.,* an α-carbon that is bound to a hydrogen, a carboxyl group, an amino group, and an R group, *e.g.*, homoserine, norleucine, methionine sulfoxide, methionine methyl sulfonium. Such analogs have modified R-groups (*e.g.*, norleucine) or modified peptide backbones, but retain the same basic chemical structure as a naturally-occurring amino acid. Amino acid mimetics refers to chemical compounds that have a structure that is different from the general chemical structure of an amino acid, but that functions in a manner similar to a naturally-occurring amino acid. Amino acids can be referred to herein by either their commonly known three letter symbols or by the one-letter symbols recommended by the IUPAC-IUB Biochemical Nomenclature Commission.

As used herein, the terms "effective amount" or "therapeutically effective amount" or "pharmaceutically effective amount" refer to a quantity sufficient to achieve a desired therapeutic and/or prophylactic effect, *e.g.,* an amount which results in the prevention of, or a decrease in, muscle dysfunction or atrophy or one or more symptoms associated therewith. In the context of therapeutic or prophylactic applications, the amount of a composition administered to the subject will depend on the type and severity of the disease and on the characteristics of the individual, such as general health, age, sex, body weight and tolerance to drugs. It will also depend on the degree, severity and type of disease. The skilled artisan will be able to determine appropriate dosages depending on these and other factors. The compositions can also be administered in combination with one or more additional therapeutic compounds. In the methods described herein, the aromatic-cationic peptides may be administered to a subject having one or more signs or symptoms of the effect associated with muscle disuse, MV implementation, and the like. For example, a "therapeutically effective amount" of one or more aromatic-cationic peptides refers to an amount sufficient to, at a minimum, ameliorate MV-induced or disuse-induced muscle atrophy, dysfunction, degradation, contractile dysfunction, damage, *etc.*

As used herein, the term "medical condition" includes, but is not limited to, any condition or disease manifested as one or more physical and/or psychological symptoms for which treatment and/or prevention is desirable, and includes previously and newly identified diseases and other disorders. For example, a medical condition may be MV-induced or disuse-induced skeletal muscle atrophy or dysfunction or contractile dysfunction or any associated symptoms or complications.

An "isolated" or "purified" polypeptide or peptide is substantially free of cellular material or other contaminating polypeptides from the cell or tissue source from which the agent is derived, or substantially free from chemical precursors or other chemicals when chemically synthesized. For example, an isolated aromatic-cationic peptide would be free of materials that would interfere with diagnostic or therapeutic uses of the agent. Such interfering materials may include enzymes, hormones and other proteinaceous and nonproteinaceous solutes.

As used herein, the term "net charge" refers to the balance of the number of positive charges and the number of negative charges carried by the amino acids present in the peptide. In this specification, it is understood that net charges are measured at physiological pH. The naturally occurring amino acids that are positively charged at physiological pH include L-lysine, L-arginine, and L-histidine. The naturally occurring amino acids that are negatively charged at physiological pH include L-aspartic acid and L-glutamic acid.

As used herein, the terms "polypeptide," "peptide," and "protein" are used interchangeably herein to mean a polymer comprising two or more amino acids joined to each other by peptide bonds or modified peptide bonds, *i.e.,* peptide isosteres. Polypeptide refers to both short chains, commonly referred to as peptides, glycopeptides or oligomers, and to longer chains, generally referred to as proteins. Polypeptides may contain amino acids other than the 20 gene-encoded amino acids. Polypeptides include amino acid sequences modified either by natural processes, such as post-translational processing, or by chemical modification techniques that are well known in the art.

As used herein, "prevention" or "preventing" of a disorder or condition refers to a compound that, in a statistical sample, reduces the occurrence of the disorder or condition in the treated sample relative to an untreated control sample, or delays the onset or reduces the severity of one or more symptoms of the disorder or condition relative to the untreated control sample. As used herein, preventing skeletal muscle dysfunction includes preventing the initiation of skeletal muscle dysfunction, delaying the initiation of skeletal muscle dysfunction, preventing the progression or advancement of skeletal muscle dysfunction, slowing the progression or advancement of skeletal muscle dysfunction, delaying the progression or advancement of skeletal muscle dysfunction, and reversing the progression of skeletal muscle dysfunction from an advanced to a less advanced stage.

As used herein, the terms "prolonged" or "prolonged-MV" or "prolonged-disuse" in reference to the cause or correlation with muscle weakness or muscle dysfunction or muscle atrophy, includes a time from at least about 0.5, 1, 2, 3, 4, 5, 6, 7, 8, 9, 10, 15, 20, 30, 50, or 100 hours, to from at least about 1, 10, 20, 50, 75, 100 or greater hours, days, or years.

As used herein, the term "simultaneous" therapeutic use refers to the administration of at least two active ingredients by the same route and at the same time or at substantially the same time.

As used herein, the term "separate" therapeutic use refers to an administration of at least two active ingredients at the same time or at substantially the same time by different routes.

The term "overlapping" therapeutic use refers to administration of one or more active ingredients at different but overlapping times. Overlapping therapeutic use includes administration of active ingredients by different routes or by the same route.

As used herein, the term "sequential" therapeutic use refers to administration of at least two active ingredients at different times, the administration route being identical or different. More particularly, sequential use refers to the whole administration of one of the active ingredients before administration of the other or others commences. It is thus possible to administer one of the active ingredients over several minutes, hours, or days before administering the other active ingredient or ingredients. There is no simultaneous treatment in this case.

As used herein, the term "subject" refers to a member of any vertebrate species. The methods of the presently disclosed subject matter are particularly useful for warm-blooded vertebrates. Provided herein is the treatment of mammals such as humans, as well as those mammals of importance due to being endangered, of economic importance (animals raised on farms for consumption by humans) and/or social importance (animals kept as pets or in zoos) to humans. In particular embodiments, the subject is a human.

As used herein, the term "muscle infirmity" refers to reduced or aberrant muscle function and includes, for example, one or more of muscle weakness, muscle dysfunction, atrophy, disuse, degradation, contractile dysfunction or damage. One example of muscle infirmity is mechanical ventilation (MV)-induced diaphragm weakness. Another example of muscle infirmity is muscle weakness induced by muscle disuse, such as by casting a limb. Muscle infirmity can be induced, derived or develop for one or more of several reasons, including but not limited to age, genetics, disease (*e.g*., infection), mechanical or chemical causes. Some non-limiting examples in which muscle infirmity arises include aging, prolonged bed rest, muscle weakness associated with microgravity (*e.g*., as in space flight), drug induced muscle weakness (*e.g*., as an effect of statins, antiretrovirals and thiazolidinediones), and cachexia due to cancer or other diseases. In some instances, muscle infirmity, such as skeletal muscle infirmity, results from oxidative stress caused by the production of reactive oxygen species ("ROS") by enzymes (*e.g.*, xanthine oxidase, NADPH oxidase) and/or the mitochondria within the muscle cells themselves. Such ROS may be produced under any number of circumstances, including those listed above. Muscle infirmity or the extent of muscle infirmity can be determined by evaluating one more physical and/or physiological parameters.

As used herein, the terms "treating" or "treatment" or "alleviation" refers to therapeutic treatment, wherein the object is to prevent or slow down (lessen) the targeted pathologic condition or disorder. A subject is successfully "treated" for MV-induced or disuse-induced muscle infirmity, if after receiving a therapeutic amount of the aromatic-cationic peptides according to the methods described herein, the subject shows observable and/or measurable reduction in or absence of one or more signs and symptoms of MV-induced or disuse-induced infirmity, such as, *e.g.*, MV-induced or disuse-induced muscle atrophy, dysfunction, degradation, contractile dysfunction, damage, and the like. It is also to be appreciated that the various modes of treatment or prevention of medical conditions as described are intended to mean "substantial," which includes total but also less than total treatment or prevention, and wherein some biologically or medically relevant result is achieved. Treating muscle infirmity, as used herein, also refers to treating any one or more of muscle dysfunction, atrophy, disuse, degradation, contractile dysfunction, damage, *etc.*

### I. Aromatic-Cationic Peptides

In one aspect, compositions and methods for the treatment or prevention of skeletal muscle infirmity *(e.g.,* weakness, atrophy, dysfunction, *etc.)* are provided. In some embodiments, the compositions and methods include administration of certain aromatic-cationic peptides, or a pharmaceutically acceptable salt thereof, such as acetate salt or trifluoroacetate salt. The aromatic-cationic peptides are water-soluble and highly polar. Despite these properties, the peptides can readily penetrate cell membranes. The aromatic-cationic peptides typically include a minimum of three amino acids or a minimum of four amino acids, covalently joined by peptide bonds. The maximum number of amino acids present in the aromatic-cationic peptides is about twenty amino acids covalently joined by peptide bonds. Suitably, the maximum number of amino acids is about twelve, more preferably about nine, and most preferably about six.

The amino acids of the aromatic-cationic peptides can be any amino acid. As used herein, the term "amino acid" is used to refer to any organic molecule that contains at least one amino group and at least one carboxyl group. Typically, at least one amino group is at the α position relative to a carboxyl group. The amino acids may be naturally occurring. Naturally occurring amino acids include, for example, the twenty most common levorotatory (L) amino acids normally found in mammalian proteins, *i.e.,* alanine (Ala), arginine (Arg), asparagine (Asn), aspartic acid (Asp), cysteine (Cys), glutamine (Gln), glutamic acid (Glu), glycine (Gly), histidine (His), isoleucine (Ile), leucine (Leu), lysine (Lys), methionine (Met), phenylalanine (Phe), proline (Pro), serine (Ser), threonine (Thr), tryptophan, (Trp), tyrosine (Tyr), and valine (Val). Other naturally occurring amino acids include, for example, amino acids that are synthesized in metabolic processes not associated with protein synthesis. For example, the amino acids ornithine and citrulline are synthesized in mammalian metabolism during the production of urea. Another example of a naturally occurring amino acid includes hydroxyproline (Hyp).

The peptides optionally contain one or more non-naturally occurring amino acids. In some embodiments, the peptide has no amino acids that are naturally occurring. The non-naturally occurring amino acids may be levorotary (L-), dextrorotatory (D-), or mixtures thereof. Non-naturally occurring amino acids are those amino acids that typically are not synthesized in normal metabolic processes in living organisms, and do not naturally occur in proteins. In addition, the non-naturally occurring amino acids suitably are also not recognized by common proteases. The non-naturally occurring amino acid can be present at any position in the peptide. For example, the non-naturally occurring amino acid can be at the N-terminus, the C-terminus, or at any position between the N-terminus and the C-terminus. Pharmaceutically acceptable salts forms of the peptides of the present technology are useful in the methods provided by the present technology as described herein (e.g., but not limited to, acetate salts or trifluoroacetate salts thereof).

The non-natural amino acids may, for example, comprise alkyl, aryl, or alkylaryl groups not found in natural amino acids. Some examples of non-natural alkyl amino acids include α-aminobutyric acid, β-aminobutyric acid, γ-aminobutyric acid, δ-aminovaleric acid, and ε-aminocaproic acid. Some examples of non-natural aryl amino acids include ortho, meta, and para-aminobenzoic acid. Some examples of non-natural alkylaryl amino acids include ortho-, meta-, and para-aminophenylacetic acid, and γ-phenyl-β-aminobutyric acid. Non-naturally occurring amino acids include derivatives of naturally occurring amino acids. The derivatives of naturally occurring amino acids may, for example, include the addition of one or more chemical groups to the naturally occurring amino acid.

For example, one or more chemical groups can be added to one or more of the 2', 3', 4', 5', or 6' position of the aromatic ring of a phenylalanine or tyrosine residue, or the 4', 5', 6', or 7' position of the benzo ring of a tryptophan residue. The group can be any chemical group that can be added to an aromatic ring. Some examples of such groups include branched or unbranched C₁-C₄ alkyl, such as methyl, ethyl, n-propyl, isopropyl, butyl, isobutyl, or t-butyl, C₁-C₄ alkyloxy *(i.e.,* alkoxy), amino, C₁-C₄ alkylamino and C₁-C₄ dialkylamino *(e.g.,* methylamino, dimethylamino), nitro, hydroxyl, halo *(i.e.,* fluoro, chloro, bromo, or iodo). Some specific examples of non-naturally occurring derivatives of naturally occurring amino acids include norvaline (Nva) and norleucine (Nle).

Another example of a modification of an amino acid in a peptide is the derivatization of a carboxyl group of an aspartic acid or a glutamic acid residue of the peptide. One example of derivatization is amidation with ammonia or with a primary or secondary amine, *e.g.* methylamine, ethylamine, dimethylamine or diethylamine. Another example of derivatization includes esterification with, for example, methyl or ethyl alcohol. Another such modification includes derivatization of an amino group of a lysine, arginine, or histidine residue. For example, such amino groups can be acylated. Some suitable acyl groups include, for example, a benzoyl group or an alkanoyl group comprising any of the C₁-C₄ alkyl groups mentioned above, such as an acetyl or propionyl group.

The non-naturally occurring amino acids are suitably resistant or insensitive to common proteases. Examples of non-naturally occurring amino acids that are resistant or insensitive to proteases include the dextrorotatory (D-) form of any of the above-mentioned naturally occurring L-amino acids, as well as L- and/or D- non-naturally occurring amino acids. The D-amino acids do not normally occur in proteins, although they are found in certain peptide antibiotics that are synthesized by means other than the normal ribosomal protein synthetic machinery of the cell. As used herein, the D-amino acids are considered to be non-naturally occurring amino acids.

In order to minimize protease sensitivity, the peptides should have less than five, preferably less than four, more preferably less than three, and most preferably, less than two contiguous L-amino acids recognized by common proteases, irrespective of whether the amino acids are naturally or non-naturally occurring. Optimally, the peptide has only D-amino acids, and no L-amino acids. If the peptide contains protease sensitive sequences of amino acids, at least one of the amino acids is preferably a non-naturally-occurring D-amino acid, thereby conferring protease resistance. An example of a protease sensitive sequence includes two or more contiguous basic amino acids that are readily cleaved by common proteases, such as endopeptidases and trypsin. Examples of basic amino acids include arginine, lysine and histidine.

The aromatic-cationic peptides should have a minimum number of net positive charges at physiological pH in comparison to the total number of amino acid residues in the peptide. The minimum number of net positive charges at physiological pH will be referred to below as (pₘ). The total number of amino acid residues in the peptide will be referred to below as (r). The minimum number of net positive charges discussed below are all at physiological pH. The term "physiological pH" as used herein refers to the normal pH in the cells of the tissues and organs of the mammalian body. For instance, the physiological pH of a human is normally approximately 7.4, but normal physiological pH in mammals may be any pH from about 7.0 to about 7.8.

Typically, a peptide has a positively charged N-terminal amino group and a negatively charged C-terminal carboxyl group. The charges cancel each other out at physiological pH. As an example of calculating net charge, the peptide Tyr-Arg-Phe-Lys-Glu-His-Trp-D-Arg has one negatively charged amino acid *(i. e.,* Glu) and four positively charged amino acids *(i.e.,* two Arg residues, one Lys, and one His). Therefore, the above peptide has a net positive charge of three.

In one embodiment, the aromatic-cationic peptides have a relationship between the minimum number of net positive charges at physiological pH (pₘ) and the total number of amino acid residues (r) wherein 3pₘ is the largest number that is less than or equal to r + 1. In this embodiment, the relationship between the minimum number of net positive charges (pₘ) and the total number of amino acid residues (r) is as follows:

**TABLE 1. Amino acid number and net positive charges (3pₘ ≤ p+1)**

| | | | | | | | | | | | | | | | | | | |
|---|---|---|---|---|---|---|---|---|---|---|---|---|---|---|---|---|---|---|
| **(r)** | 3 | 4 | 5 | 6 | 7 | 8 | 9 | 10 | 11 | 12 | 13 | 14 | 15 | 16 | 17 | 18 | 19 | 20 |
| **(pₘ)** | 1 | 1 | 2 | 2 | 2 | 3 | 3 | 3 | 4 | 4 | 4 | 5 | 5 | 5 | 6 | 6 | 6 | 7 |

In another embodiment, the aromatic-cationic peptides have a relationship between the minimum number of net positive charges (pₘ) and the total number of amino acid residues (r) wherein 2pₘ is the largest number that is less than or equal to r + 1. In this embodiment, the relationship between the minimum number of net positive charges (pₘ) and the total number of amino acid residues (r) is as follows:

**TABLE 2. Amino acid number and net positive charges (2pₘ ≤ p+1)**

| | | | | | | | | | | | | | | | | | | |
|---|---|---|---|---|---|---|---|---|---|---|---|---|---|---|---|---|---|---|
| **(r)** | 3 | 4 | 5 | 6 | 7 | 8 | 9 | 10 | 11 | 12 | 13 | 14 | 15 | 16 | 17 | 18 | 19 | 20 |
| **(pₘ)** | 2 | 2 | 3 | 3 | 4 | 4 | 5 | 5 | 6 | 6 | 7 | 7 | 8 | 8 | 9 | 9 | 10 | 10 |

In one embodiment, the minimum number of net positive charges (pₘ) and the total number of amino acid residues (r) are equal. In another embodiment, the peptides have three or four amino acid residues and a minimum of one net positive charge, suitably, a minimum of two net positive charges and more preferably a minimum of three net positive charges.

It is also important that the aromatic-cationic peptides have a minimum number of aromatic groups in comparison to the total number of net positive charges (pₜ). The minimum number of aromatic groups will be referred to below as (a). Naturally occurring amino acids that have an aromatic group include the amino acids histidine, tryptophan, tyrosine, and phenylalanine. For example, the hexapeptide Lys-Gln-Tyr-D-Arg-Phe-Trp has a net positive charge of two (contributed by the lysine and arginine residues) and three aromatic groups (contributed by tyrosine, phenylalanine and tryptophan residues).

The aromatic-cationic peptides should also have a relationship between the minimum number of aromatic groups (a) and the total number of net positive charges at physiological pH (pₜ) wherein 3a is the largest number that is less than or equal to pₜ + 1, except that when pₜ is 1, a may also be 1. In this embodiment, the relationship between the minimum number of aromatic groups (a) and the total number of net positive charges (pₜ) is as follows:

**TABLE 3. Aromatic groups and net positive charges (3a ≤ pₜ+1 or a= pₜ=1)**

| | | | | | | | | | | | | | | | | | | | | |
|---|---|---|---|---|---|---|---|---|---|---|---|---|---|---|---|---|---|---|---|---|
| **(pₜ)** | 1 | 2 | 3 | 4 | 5 | 6 | 7 | 8 | 9 | 10 | 11 | 12 | 13 | 14 | 15 | 16 | 17 | 18 | 19 | 20 |
| **(a)** | 1 | 1 | 1 | 1 | 2 | 2 | 2 | 3 | 3 | 3 | 4 | 4 | 4 | 5 | 5 | 5 | 6 | 6 | 6 | 7 |

In another embodiment, the aromatic-cationic peptides have a relationship between the minimum number of aromatic groups (a) and the total number of net positive charges (pₜ) wherein 2a is the largest number that is less than or equal to pₜ + 1. In this embodiment, the relationship between the minimum number of aromatic amino acid residues (a) and the total number of net positive charges (pₜ) is as follows:

**TABLE 4. Aromatic groups and net positive charges (2a ≤ pₜ+1 or a= pₜ=1)**

| | | | | | | | | | | | | | | | | | | | | |
|---|---|---|---|---|---|---|---|---|---|---|---|---|---|---|---|---|---|---|---|---|
| **(pₜ)** | 1 | 2 | 3 | 4 | 5 | 6 | 7 | 8 | 9 | 10 | 11 | 12 | 13 | 14 | 15 | 16 | 17 | 18 | 19 | 20 |
| **(a)** | 1 | 1 | 2 | 2 | 3 | 3 | 4 | 4 | 5 | 5 | 6 | 6 | 7 | 7 | 8 | 8 | 9 | 9 | 10 | 10 |

In another embodiment, the number of aromatic groups (a) and the total number of net positive charges (pₜ) are equal.

Carboxyl groups, especially the terminal carboxyl group of a C-terminal amino acid, are suitably amidated with, for example, ammonia to form the C-terminal amide. Alternatively, the terminal carboxyl group of the C-terminal amino acid may be amidated with any primary or secondary amine. The primary or secondary amine may, for example, be an alkyl, especially a branched or unbranched C₁-C₄ alkyl, or an aryl amine. Accordingly, the amino acid at the C-terminus of the peptide may be converted to an amido, N-methylamido, N-ethylamido, N,N-dimethylamido, N,N-diethylamido, N-methyl-N-ethylamido, N-phenylamido or N-phenyl-N-ethylamido group. The free carboxylate groups of the asparagine, glutamine, aspartic acid, and glutamic acid residues not occurring at the C-terminus of the aromatic-cationic peptides may also be amidated wherever they occur within the peptide. The amidation at these internal positions may be with ammonia or any of the primary or secondary amines described above.

In one embodiment, the aromatic-cationic peptide is a tripeptide having two net positive charges and at least one aromatic amino acid. In a particular embodiment, the aromatic-cationic peptide is a tripeptide having two net positive charges and two aromatic amino acids.

Aromatic-cationic peptides include, but are not limited to, the following peptide examples:
Lys-D-Arg-Tyr-NH₂
Phe-D-Arg-His
D-Tyr-Trp-Lys-NH₂
Trp-D-Lys-Tyr-Arg-NH₂
Tyr-His-D-Gly-Met
Phe-Arg-D-His-Asp
Tyr-D-Arg-Phe-Lys-Glu-NH₂
Met-Tyr-D-Lys-Phe-Arg
D-His-Glu-Lys-Tyr-D-Phe-Arg
Lys-D-Gln-Tyr-Arg-D-Phe-Trp-NH₂
Phe-D-Arg-Lys-Trp-Tyr-D-Arg-His
Gly-D-Phe-Lys-Tyr-His-D-Arg-Tyr-NH₂
Val-D-Lys-His-Tyr-D-Phe-Ser-Tyr-Arg-NH₂
Trp-Lys-Phe-D-Asp-Arg-Tyr-D-His-Lys
Lys-Trp-D-Tyr-Arg-Asn-Phe-Tyr-D-His-NH₂
Thr-Gly-Tyr-Arg-D-His-Phe-Trp-D-His-Lys
Asp-D-Trp-Lys-Tyr-D-His-Phe-Arg- D-Gly-Lys-NH₂
D-His-Lys-Tyr- D-Phe-Glu- D-Asp- D-His- D-Lys-Arg-Trp-NH₂
Ala-D-Phe-D-Arg-Tyr-Lys-D-Trp-His-D-Tyr-Gly-Phe
Tyr-D-His-Phe- D-Arg-Asp-Lys- D-Arg-His-Trp-D-His-Phe
Phe-Phe-D-Tyr-Arg-Glu-Asp-D-Lys-Arg-D-Arg-His-Phe-NH₂
Phe-Try-Lys-D-Arg-Trp-His-D-Lys-D-Lys-Glu-Arg-D-Tyr-Thr
Tyr-Asp-D-Lys-Tyr-Phe- D-Lys- D-Arg-Phe-Pro-D-Tyr-His-Lys
Glu-Arg-D-Lys-Tyr- D-Val-Phe- D-His-Trp-Arg-D-Gly-Tyr-Arg-D-Met-NH2
Arg-D-Leu-D-Tyr-Phe-Lys-Glu- D-Lys-Arg-D-Trp-Lys- D-Phe-Tyr-D-Arg-Gly
D-Glu-Asp-Lys-D-Arg-D-His-Phe-Phe-D-Val-Tyr-Arg-Tyr-D-Tyr-Arg-His-Phe-NH₂
Asp-Arg-D-Phe-Cys-Phe-D-Arg-D-Lys-Tyr-Arg-D-Tyr-Trp-D-His-Tyr-D-Phe-Lys-Phe
His-Tyr-D-Arg-Trp-Lys-Phe-D-Asp-Ala-Arg-Cys-D-Tyr-His-Phe-D-Lys-Tyr-His-Ser-NH₂

Gly-Ala-Lys-Phe-D-Lys-Glu-Arg-Tyr-His-D-Arg-D-Arg-Asp-Tyr-Trp-D-His-Trp-His-D-
Lys-Asp

Thr-Tyr-Arg-D-Lys-Trp-Tyr-Glu-Asp-D-Lys-D-Arg-His-Phe-D-Tyr-Gly-Val-Ile-D-His-
Arg-Tyr-Lys-NH2

In one embodiment, the peptides have mu-opioid receptor agonist activity (*i.e*., they activate the mu-opioid receptor). Peptides which have mu-opioid receptor agonist activity are typically those peptides which have a tyrosine residue or a tyrosine derivative at the N-terminus (*i.e*., the first amino acid position). Suitable derivatives of tyrosine include 2'-methyltyrosine (Mmt); 2',6'-dimethyltyrosine (2'6'-Dmt); 3',5'-dimethyltyrosine (3'5'Dmt); N,2',6'-trimethyltyrosine (Tmt); and 2'-hydroxy-6'-methyltryosine (Hmt).

In one embodiment, a peptide that has mu-opioid receptor agonist activity has the formula Tyr-D-Arg-Phe-Lys-NH₂ (referred to herein as "SS-01"). SS-01 has a net positive charge of three, contributed by the amino acids tyrosine, arginine, and lysine and has two aromatic groups contributed by the amino acids phenylalanine and tyrosine. The tyrosine of SS-01 can be a modified derivative of tyrosine such as in 2',6'-dimethyltyrosine to produce the compound having the formula 2',6'-Dmt-D-Arg-Phe-Lys-NH₂ (referred to herein as "SS-02"). SS-02 has a molecular weight of 640 and carries a net three positive charge at physiological pH. SS-02 readily penetrates the plasma membrane of several mammalian cell types in an energy-independent manner (Zhao et al., J. Pharmacol Exp Ther., 304:425-432, 2003).

Alternatively, in other instances, the aromatic-cationic peptide does not have mu-opioid receptor agonist activity. For example, during long-term treatment, such as in a chronic disease state or condition, the use of an aromatic-cationic peptide that activates the mu-opioid receptor may be contraindicated. In these instances, the potentially adverse or addictive effects of the aromatic-cationic peptide may preclude the use of an aromatic-cationic peptide that activates the mu-opioid receptor in the treatment regimen of a human patient or other mammal. Potential adverse effects may include sedation, constipation and respiratory depression. In such instances an aromatic-cationic peptide that does not activate the mu-opioid receptor may be an appropriate treatment. Peptides that do not have mu-opioid receptor agonist activity generally do not have a tyrosine residue or a derivative of tyrosine at the N-terminus *(i.e.,* amino acid position 1). The amino acid at the N-terminus can be any naturally occurring or non-naturally occurring amino acid other than tyrosine. In one embodiment, the amino acid at the N-terminus is phenylalanine or its derivative. Exemplary derivatives of phenylalanine include 2'-methylphenylalanine (Mmp), 2',6'-dimethylphenylalanine (2',6'-Dmp), N,2',6'-trimethylphenylalanine (Tmp), and 2'-hydroxy-6'-methylphenylalanine (Hmp).

An example of an aromatic-cationic peptide that does not have mu-opioid receptor agonist activity has the formula Phe-D-Arg-Phe-Lys-NH₂ (referred to herein as "SS-20"). Alternatively, the N-terminal phenylalanine can be a derivative of phenylalanine such as 2',6'-dimethylphenylalanine (2'6'-Dmp). SS-01 containing 2',6'-dimethylphenylalanine at amino acid position 1 has the formula 2',6'-Dmp-D-Arg-Phe-Lys-NH₂. In one embodiment, the amino acid sequence of SS-02 is rearranged such that Dmt is not at the N-terminus. An example of such an aromatic-cationic peptide that does not have mu-opioid receptor agonist activity has the formula D-Arg-2'6'-Dmt-Lys-Phe-NH₂.

Suitable substitution variants of the peptides listed herein include conservative amino acid substitutions. Amino acids may be grouped according to their physicochemical characteristics as follows:
(a) Non-polar amino acids: Ala(A) Ser(S) Thr(T) Pro(P) Gly(G) Cys (C);
(b) Acidic amino acids: Asn(N) Asp(D) Glu(E) Gln(Q);
(c) Basic amino acids: His(H) Arg(R) Lys(K);
(d) Hydrophobic amino acids: Met(M) Leu(L) Ile(I) Val(V); and
(e) Aromatic amino acids: Phe(F) Tyr(Y) Trp(W) His (H).

Substitutions of an amino acid in a peptide by another amino acid in the same group is referred to as a conservative substitution and may preserve the physicochemical characteristics of the original peptide. In contrast, substitutions of an amino acid in a peptide by another amino acid in a different group is generally more likely to alter the characteristics of the original peptide.

Examples of peptides that activate mu-opioid receptors include, but are not limited to, the aromatic-cationic peptides shown in Table 5.

**TABLE 5. Peptide Analogs with Mu-Opioid Activity**

| **Amino Acid Position 1** | **Amino Acid Position 2** | **Amino Acid Position 3** | **Amino Acid Position 4** | C-**Terminal Modification** |
|---|---|---|---|---|
| Tyr | D-Arg | Phe | Lys | **NH₂** |
| Tyr | D-Arg | Phe | Orn | NH₂ |
| Tyr | D-Arg | Phe | Dab | NH₂ |
| Tyr | D-Arg | Phe | Dap | NH₂ |
| 2'6'Dmt | D-Arg | Phe | Lys | NH₂ |
| 2'6'Dmt | D-Arg | Phe | Lys-NH(CH₂)₂-NH-dns | NH₂ |
| 2'6'Dmt | D-Arg | Phe | Lys-NH(CH₂)₂-NH-atn | NH₂ |
| 2'6'Dmt | D-Arg | Phe | dnsLys | NH₂ |
| 2'6'Dmt | D-Cit | Phe | Lys | NH₂ |
| 2'6'Dmt | D-Cit | Phe | Ahp | NH₂ |
| 2'6'Dmt | D-Arg | Phe | Orn | NH₂ |
| 2'6'Dmt | D-Arg | Phe | Dab | NH₂ |
| 2'6'Dmt | D-Arg | Phe | Dap | NH₂ |
| 2'6'Dmt | D-Arg | Phe | Ahp(2-aminoheptanoic acid) | NH₂ |
| Bio-2'6'Dmt | D-Arg | Phe | Lys | NH₂ |
| 3'5'Dmt | D-Arg | Phe | Lys | NH₂ |
| 3'5'Dmt | D-Arg | Phe | Orn | NH₂ |
| 3'5'Dmt | D-Arg | Phe | Dab | NH₂ |
| 3'5'Dmt | D-Arg | Phe | Dap | NH₂ |
| Tyr | D-Arg | Tyr | Lys | NH₂ |
| Tyr | D-Arg | Tyr | Orn | NH₂ |
| Tyr | D-Arg | Tyr | Dab | NH₂ |
| Tyr | D-Arg | Tyr | Dap | NH₂ |
| 2'6'Dmt | D-Arg | Tyr | Lys | NH₂ |
| 2'6'Dmt | D-Arg | Tyr | Orn | NH₂ |
| 2'6'Dmt | D-Arg | Tyr | Dab | NH₂ |
| 2'6'Dmt | D-Arg | Tyr | Dap | NH₂ |
| 2'6'Dmt | D-Arg | 2'6'Dmt | Lys | NH₂ |
| 2'6'Dmt | D-Arg | 2'6'Dmt | Orn | NH₂ |
| 2'6'Dmt | D-Arg | 2'6'Dmt | Dab | NH₂ |
| 2'6'Dmt | D-Arg | 2'6'Dmt | Dap | NH₂ |
| 3'5'Dmt | D-Arg | 3'5'Dmt | Arg | NH₂ |
| 3'5'Dmt | D-Arg | 3'5'Dmt | Lys | NH₂ |
| 3'5'Dmt | D-Arg | 3'5'Dmt | Orn | NH₂ |
| 3'5'Dmt | D-Arg | 3'5'Dmt | Dab | NH₂ |
| Tyr | D-Lys | Phe | Dap | NH₂ |
| Tyr | D-Lys | Phe | Arg | NH₂ |
| Tyr | D-Lys | Phe | Lys | NH₂ |
| Tyr | D-Lys | Phe | Orn | NH₂ |
| 2'6'Dmt | D-Lys | Phe | Dab | NH₂ |
| 2'6'Dmt | D-Lys | Phe | Dap | NH₂ |
| 2'6'Dmt | D-Lys | Phe | Arg | NH₂ |
| 2'6'Dmt | D-Lys | Phe | Lys | NH₂ |
| 3'5'Dmt | D-Lys | Phe | Orn | NH₂ |
| 3'5'Dmt | D-Lys | Phe | Dab | NH₂ |
| 3'5'Dmt | D-Lys | Phe | Dap | NH₂ |
| 3'5'Dmt | D-Lys | Phe | Arg | NH₂ |
| Tyr | D-Lys | Tyr | Lys | NH₂ |
| Tyr | D-Lys | Tyr | Orn | NH₂ |
| Tyr | D-Lys | Tyr | Dab | NH₂ |
| Tyr | D-Lys | Tyr | Dap | NH₂ |
| 2'6'Dmt | D-Lys | Tyr | Lys | NH₂ |
| 2'6'Dmt | D-Lys | Tyr | Orn | NH₂ |
| 2'6'Dmt | D-Lys | Tyr | Dab | NH₂ |
| 2'6'Dmt | D-Lys | Tyr | Dap | NH₂ |
| 2'6'Dmt | D-Lys | 2'6'Dmt | Lys | NH₂ |
| 2'6'Dmt | D-Lys | 2'6'Dmt | Orn | NH₂ |
| 2'6'Dmt | D-Lys | 2'6'Dmt | Dab | NH₂ |
| 2'6'Dmt | D-Lys | 2'6'Dmt | Dap | NH₂ |
| 2'6'Dmt | D-Arg | Phe | dnsDap | NH₂ |
| 2'6'Dmt | D-Arg | Phe | atnDap | NH₂ |
| 3'5'Dmt | D-Lys | 3'5'Dmt | Lys | NH₂ |
| 3'5'Dmt | D-Lys | 3'5'Dmt | Orn | NH₂ |
| 3'5'Dmt | D-Lys | 3'5'Dmt | Dab | NH₂ |
| 3'5'Dmt | D-Lys | 3'5'Dmt | Dap | NH₂ |
| Tyr | D-Lys | Phe | Arg | NH₂ |
| Tyr | D-Orn | Phe | Arg | NH₂ |
| Tyr | D-Dab | Phe | Arg | NH₂ |
| Tyr | D-Dap | Phe | Arg | NH₂ |
| 2'6'Dmt | D-Arg | Phe | Arg | NH₂ |
| 2'6'Dmt | D-Lys | Phe | Arg | NH₂ |
| 2'6'Dmt | D-Orn | Phe | Arg | NH₂ |
| 2'6'Dmt | D-Dab | Phe | Arg | NH₂ |
| 3'5'Dmt | D-Dap | Phe | Arg | NH₂ |
| 3'5'Dmt | D-Arg | Phe | Arg | NH₂ |
| 3'5'Dmt | D-Lys | Phe | Arg | NH₂ |
| 3'5'Dmt | D-Orn | Phe | Arg | NH₂ |
| Tyr | D-Lys | Tyr | Arg | NH₂ |
| Tyr | D-Orn | Tyr | Arg | NH₂ |
| Tyr | D-Dab | Tyr | Arg | NH₂ |
| Tyr | D-Dap | Tyr | Arg | NH₂ |
| 2'6'Dmt | D-Arg | 2'6'Dmt | Arg | NH₂ |
| 2'6'Dmt | D-Lys | 2'6'Dmt | Arg | NH₂ |
| 2'6'Dmt | D-Orn | 2'6'Dmt | Arg | NH₂ |
| 2'6'Dmt | D-Dab | 2'6'Dmt | Arg | NH₂ |
| 3'5'Dmt | D-Dap | 3'5'Dmt | Arg | NH₂ |
| 3'5'Dmt | D-Arg | 3'5'Dmt | Arg | NH₂ |
| 3'5'Dmt | D-Lys | 3'5'Dmt | Arg | NH₂ |
| 3'5'Dmt | D-Orn | 3'5'Dmt | Arg | NH₂ |
| Mmt | D-Arg | Phe | Lys | NH₂ |
| Mmt | D-Arg | Phe | Orn | NH₂ |
| Mmt | D-Arg | Phe | Dab | NH₂ |
| Mmt | D-Arg | Phe | Dap | NH₂ |
| Tmt | D-Arg | Phe | Lys | NH₂ |
| Tmt | D-Arg | Phe | Orn | NH₂ |
| Tmt | D-Arg | Phe | Dab | NH₂ |
| Tmt | D-Arg | Phe | Dap | NH₂ |
| Hmt | D-Arg | Phe | Lys | NH₂ |
| Hmt | D-Arg | Phe | Orn | NH₂ |
| Hmt | D-Arg | Phe | Dab | NH₂ |
| Hmt | D-Arg | Phe | Dap | NH₂ |
| Mmt | D-Lys | Phe | Lys | NH₂ |
| Mmt | D-Lys | Phe | Orn | NH₂ |
| Mmt | D-Lys | Phe | Dab | NH₂ |
| Mmt | D-Lys | Phe | Dap | NH₂ |
| Mmt | D-Lys | Phe | Arg | NH₂ |
| Tmt | D-Lys | Phe | Lys | NH₂ |
| Tmt | D-Lys | Phe | Orn | NH₂ |
| Tmt | D-Lys | Phe | Dab | NH₂ |
| Tmt | D-Lys | Phe | Dap | NH₂ |
| Tmt | D-Lys | Phe | Arg | NH₂ |
| Hmt | D-Lys | Phe | Lys | NH₂ |
| Hmt | D-Lys | Phe | Orn | NH₂ |
| Hmt | D-Lys | Phe | Dab | NH₂ |
| Hmt | D-Lys | Phe | Dap | NH₂ |
| Hmt | D-Lys | Phe | Arg | NH₂ |
| Mmt | D-Lys | Phe | Arg | NH₂ |
| Mmt | D-Orn | Phe | Arg | NH₂ |
| Mmt | D-Dab | Phe | Arg | NH₂ |
| Mmt | D-Dap | Phe | Arg | NH₂ |
| Mmt | D-Arg | Phe | Arg | NH₂ |
| Tmt | D-Lys | Phe | Arg | NH₂ |
| Tmt | D-Orn | Phe | Arg | NH₂ |
| Tmt | D-Dab | Phe | Arg | NH₂ |
| Tmt | D-Dap | Phe | Arg | NH₂ |
| Tmt | D-Arg | Phe | Arg | NH₂ |
| Hmt | D-Lys | Phe | Arg | NH₂ |
| Hmt | D-Orn | Phe | Arg | NH₂ |
| Hmt | D-Dab | Phe | Arg | NH₂ |
| Hmt | D-Dap | Phe | Arg | NH₂ |
| Hmt | D-Arg | Phe | Arg | NH₂ |

| | | | | |
|---|---|---|---|---|
| Cha = cyclohexyl alanine Dab = diaminobutyric Dap = diaminopropionic acid Dmt = dimethyltyrosine Mmt = 2'-methyltyrosine Tmt = N, 2',6'-trimethyltyrosine Hmt = 2'-hydroxy,6'-methyltyrosine dnsDap = β-dansyl-L-α,β-diaminopropionic acid atnDap = β-anthraniloyl-L-α,β-diaminopropionic acid Bio = biotin | | | | |

Examples of peptides that do not activate mu-opioid receptors include, but are not limited to, the aromatic-cationic peptides shown in Table 6.

**TABLE 6. Peptide Analogs Lacking Mu-Opioid Activity**

| **Amino Acid Position 1** | **Amino Acid Position 2** | **Amino Acid Position 3** | **Amino Acid Position 4** | **C-Terminal Modification** |
|---|---|---|---|---|
| D-Arg | Dmt | Lys | Phe | NH₂ |
| D-Arg | Dmt | Phe | Lys | NH₂ |
| D-Arg | Phe | Lys | Dmt | NH₂ |
| D-Arg | Phe | Dmt | Lys | NH₂ |
| D-Arg | Lys | Dmt | Phe | NH₂ |
| D-Arg | Lys | Phe | Dmt | NH₂ |
| Phe | Lys | Dmt | D-Arg | NH₂ |
| Phe | Lys | D-Arg | Dmt | NH₂ |
| Phe | D-Arg | Phe | Lys | NH₂ |
| Phe | D-Arg | Dmt | Lys | NH₂ |
| Phe | D-Arg | Lys | Dmt | NH₂ |
| Phe | Dmt | D-Arg | Lys | NH₂ |
| Phe | Dmt | Lys | D-Arg | NH₂ |
| Lys | Phe | D-Arg | Dmt | NH₂ |
| Lys | Phe | Dmt | D-Arg | NH₂ |
| Lys | Dmt | D-Arg | Phe | NH₂ |
| Lys | Dmt | Phe | D-Arg | NH₂ |
| Lys | D-Arg | Phe | Dmt | NH₂ |
| Lys | D-Arg | Dmt | Phe | NH₂ |
| D-Arg | Dmt | D-Arg | Phe | NH₂ |
| D-Arg | Dmt | D-Arg | Dmt | NH₂ |
| D-Arg | Dmt | D-Arg | Tyr | NH₂ |
| D-Arg | Dmt | D-Arg | Trp | NH₂ |
| Trp | D-Arg | Phe | Lys | NH₂ |
| Trp | D-Arg | Tyr | Lys | NH₂ |
| Trp | D-Arg | Trp | Lys | NH₂ |
| Trp | D-Arg | Dmt | Lys | NH₂ |
| D-Arg | Trp | Lys | Phe | NH₂ |
| D-Arg | Trp | Phe | Lys | NH₂ |
| D-Arg | Trp | Lys | Dmt | NH₂ |
| D-Arg | Trp | Dmt | Lys | NH₂ |
| D-Arg | Lys | Trp | Phe | NH₂ |
| D-Arg | Lys | Trp | Dmt | NH₂ |
| Cha | D-Arg | Phe | Lys | NH₂ |
| Ala | D-Arg | Phe | Lys | NH₂ |

The amino acids of the peptides shown in Table 5 and 6 may be in either the L- or the D- configuration.

The peptides may be synthesized by any of the methods well known in the art. Suitable methods for chemically synthesizing the protein include, for example, those described by Stuart and Young in Solid Phase Peptide Synthesis, Second Edition, Pierce Chemical Company (1984), and in Methods Enzymol., 289, Academic Press, Inc, New York (1997).

### II. Use of Aromatic-Cationic Peptides

Elevated ROS emissions have been shown to be a causative agent for oxidative stress and the concomitant muscle infirmities (*e.g.*, weakness, atrophy, dysfunction) in MV-induced and disuse-induced skeletal muscle weakness. Mitochondria in the muscle cells appear to be the leading ROS producers, and as shown below in the Experimental Examples, mitochondrial ROS emissions play a role in MV-induced and disuse-induced oxidative stress that leads to skeletal muscle (*e.g.*, diaphragm, soleus and plantaris muscle) infirmities. While NADPH activation and xanthine oxidase activation also play a role in ROS production, NADPH activity is minimal (*i.e.* 5%) and inhibition of xanthine oxidase activity does not completely protect against the effects of skeletal muscle disuse-induced or MV-induced oxidative stress and the concomitant atrophy and weakness. Moreover, mitochondrial ROS emission is an up-stream signal for the MV- or disuse-induced activation of proteases, *e.g.*, calpain, caspase-3 and/or caspase-12, in the diaphragm and other skeletal muscles.

Accordingly, the present disclosure describes methods and compositions including mitochondria-targeted, antioxidant, aromatic-cationic peptides capable of reducing mitochondrial ROS production in the diaphragm during prolonged MV, or in other skeletal muscles, *e.g.*, soleus or plantaris muscle, during limb immobilization or muscle disuse in general.

In one aspect, the present disclosure provides a mitochondria-targeted antioxidant, *i.e.,* D-Arg-2',6'Dmt-Lys-Phe-NH₂ or "SS-31" or a pharmaceutically acceptable salt thereof, such as acetate salt or trifluoroacetate salt. For example, in some embodiments, SS-31 is used as a therapeutic and/or a prophylactic agent in subjects suffering from, or at risk of suffering from muscle infirmities such as weakness, atrophy, dysfunction, *etc.* caused by mitochondrial derived ROS. In some embodiments, SS-31 decreases mitochondrial ROS emission in muscle. Additionally or alternatively, in some embodiments, SS-31 selectively concentrates in the mitochondria of skeletal muscle and provides radical scavenging of H₂O₂, OH-, and ONOO-, and in some embodiments, radical scavenging is on a dose-dependent basis.

In some embodiments, methods of treating muscle infirmities (*e.g.*, weakness, atrophy, dysfunction, *etc.*) are described. In such therapeutic applications, compositions or medicaments including an aromatic cationic peptide such as SS-31 or a pharmaceutically acceptable salt thereof, such as acetate salt or trifluoroacetate salt, are administered to a subject suspected of, or already suffering from, muscle infirmity, in an amount sufficient to prevent, reduce, alleviate, or at least partially arrest, the symptoms of muscle infirmity, including its complications and intermediate pathological phenotypes in development of the infirmity. As such, the invention provides methods of treating an individual afflicted, or suspected of suffering from muscle infirmities described herein. In one embodiment, the aromatic cationic peptide SS-31, or a pharmaceutically acceptable salt thereof, such as acetate salt or trifluoroacetate salt, is administered.

In another aspect, the disclosure provides a method for preventing, or reducing the likelihood of muscle infirmity, as described herein, by administering to the subject an aromatic-cationic peptide that prevents or reduces the likelihood of the initiation or progression of the infirmity. Subjects at risk for developing muscle infirmity can be readily identified, *e.g.,* a subject preparing for or about to undergo MV or related diaphragmatic muscles disuse or any other skeletal muscle disuse that may be envisaged by a medical professional (*e.g.*, casting a limb). In one embodiment, the aromatic cationic peptide includes SS-31 or a pharmaceutically acceptable salt thereof, such as acetate salt or trifluoroacetate salt.

In such prophylactic applications, a pharmaceutical composition or medicament comprising one or more aromatic-cationic peptides or a pharmaceutically acceptable salt thereof, such as acetate salt or trifluoroacetate salt, is administered to a subject susceptible to, or otherwise at risk of muscle infirmity in an amount sufficient to eliminate or reduce the risk, lessen the severity, or delay the onset of muscle infirmity, including biochemical, histologic and/or behavioral symptoms of the infirmity, its complications and intermediate pathological phenotypes presenting during development of the infirmity. Administration of one or more of the aromatic-cationic peptide disclosed herein can occur prior to the manifestation of symptoms characteristic of the aberrancy, such that the disorder is prevented or, alternatively, delayed in its progression. The appropriate compound can be determined based on screening assays described above or as well known in the art. In one embodiment, the pharmaceutical composition includes SS-31 or a pharmaceutically acceptable salt thereof, such as acetate salt or trifluoroacetate salt.

In various embodiments, suitable *in vitro* or *in vivo* assays are performed to determine the effect of a specific aromatic-cationic peptide-based therapeutic and whether its administration is indicated for treatment. In various embodiments, assays can be performed with representative animal models, to determine if a given aromatic-cationic peptide-based therapeutic exerts the desired effect in preventing or treating muscle weakness (*e.g.*, atrophy, dysfunction, *etc.*)*.* Compounds for use in therapy can be tested in suitable animal model systems including, but not limited to rats, mice, chicken, cows, monkeys, rabbits, and the like, prior to testing in human subjects. Similarly, for *in vivo* testing, any of the animal model system known in the art can be used prior to administration to human subjects.

In some embodiments, subjects in need of protection from or treatment of muscle infirmity also include subjects suffering from a disease, condition or treatment associated with oxidative damage. Typically, the oxidative damage is caused by free radicals, such as reactive oxygen species (ROS) and/or reactive nitrogen species (RNS). Examples of ROS and RNS include hydroxyl radical (HO•), superoxide anion radical (O₂•⁻), nitric oxide (NO•), hydrogen peroxide (H₂O₂), hypochlorous acid (HOCl) and peroxynitrite anion (ONOO⁻).

Respiratory muscle infirmity may result from prolonged MV, *e.g*., greater than 12 hours. In some embodiments, the respiratory muscle infirmity is due to contractile dysfunction and/or atrophy. However, such prolonged MV is not limited to any specific time-length. For example, in some embodiments, prolonged MV includes a time from at least about 0.5, 1, 2, 3, 4, 5, 6, 7, 8, 9, 10, 15, 20, 30, 50, or 100 hours, to from at least about 1, 10, 20, 50, 75, 100 or greater hours, days, or years. In another embodiment, prolonged MV includes a time from at least about 5, 6, 7, 8, 9 or 10 hours, to from at least about 10, 20 or 50 hours. In some embodiments, prolonged MV is from about at least 10-12 hours to any time greater than the 10-12 hour period. In some embodiments, administration of the aromatic peptide compositions described herein is provided at any time during MV or muscle immobilization. In some embodiments, one or more doses of a cationic peptide composition is administered before MV, immediately after MV initiation, during MV, and/or immediately after MV.

Muscle disuse atrophy also presents an obstacle to recovery for subjects attempting to reestablishment muscle function subsequent to immobilization. In this respect, the aromatic-cationic peptides or a pharmaceutically acceptable salt thereof, such as acetate salt or trifluoroacetate salt, described herein provide for prophylactic and therapeutic methods of treating a subject having or at risk of having skeletal muscle-associated infirmities. Such muscle infirmities result from or include, but are not limited to, muscle disuse or MV, wherein the muscle disuse or MV induces apoptosis, oxidative stress, oxidative damage, contractile dysfunction, muscle atrophy, muscle proteolysis, protease activation, mitochondrial-derived ROS emission, mitochondrial H₂O₂ release, mitochondrial uncoupling, impaired mitochondria coupling, impaired state 3 mitochondrial respiration, impaired state 4 mitochondrial respiration, decreased respiratory control ration (RCR), reduced lipid peroxidation, or any combination thereof.

Composition comprising a cationic peptide disclosed herein to treat or prevent muscle infirmity associated with muscle immobilization *e.g*., due to casting or other disuse can be administered at any time before, during or after the immobilization or disuse. For example, in some embodiments, one or more doses of a cationic peptide composition is administered before muscle immobilization or disuse, immediately after muscle immobilization or disuse, during the course of muscle immobilization or disuse, and/or after muscle immobilization or disuse *(e.g*., after cast removal). By way of example, and not by way of limitation, in some embodiments, a cationic peptide *(e.g.,* SS-31 or a pharmaceutically acceptable salt thereof, such as acetate salt or trifluoroacetate salt) is administered once per day, twice per day, three times per day, four times per day six times per day or more, for the duration of the immobilization or disuse. In other embodiments, a cationic peptide *(e.g.,* SS-31 or a pharmaceutically acceptable salt thereof, such as acetate salt or trifluoroacetate salt) is administered daily, every other day, twice, three times, or for times per week, or once, twice three, four, five or six times per month for the duration of the immobilization or disuse.

In some embodiment, methods to treat or prevent muscle infirmity due to muscle disuse or disuse atrophy, associated with loss of muscle mass and strength, are also disclosed. Atrophy is a physiological process relating to the reabsorption and degradation of tissues, *e.g*., fibrous muscle tissue, which involves apoptosis at the cellular level. When atrophy occurs from loss of trophic support or other disease, it is known as pathological atrophy. Such atrophy or pathological atrophy may result from, or is related to, limb immobilization, prolonged limb immobilization, casting limb immobilization, MV, prolonged MV, extended bed rest cachexia, congestive heart failure, liver disease, sarcopenia, wasting, poor nourishment, poor circulation, hormonal irregularities, loss of nerve function, and the like. Accordingly, the present methods provide for the prevention and/or treatment of muscle infirmities, including skeletal muscle atrophy, in a subject by administering an effective amount of an aromatic-cationic peptide or a pharmaceutically acceptable salt thereof, such as acetate salt or trifluoroacetate salt to a subject in need thereof.

Additional examples of muscle infirmitites which can be treated, prevented, or alleviated by administering the compositions and formulations disclosed herein include, without limitation, age-related muscle infirmities, muscle infirmities associated with prolonged bed rest, muscle infirmities such as weakness and atrophy associated with microgravity, as in space flight, muscle infirmities associated with effects of certain drugs (e.g., statins, antiretrovirals, and thiazolidinediones (TZDs)), and muscle infirmities such as cachexia, for example cachexia caused by cancer or other diseases.

### III. Modes of Administration and Dosages

Any method known to those in the art for contacting a cell, organ or tissue with a peptide may be employed. Suitable methods include *in vitro, ex vivo, or in vivo* methods. *In vivo* methods typically include the administration of an aromatic-cationic peptide, such as those described above, to a mammal, suitably a human. When used *in vivo* for therapy, the aromatic-cationic peptides or a pharmaceutically acceptable salt thereof, such as acetate salt or trifluoroacetate salt are administered to the subject in effective amounts (*i.e.,* amounts that have desired therapeutic effect). The dose and dosage regimen will depend upon the degree of the muscle infirmity in the subject, the characteristics of the particular aromatic-cationic peptide used, *e.g.,* its therapeutic index, the subject, and the subject's history.

The effective amount may be determined during pre-clinical trials and clinical trials by methods familiar to physicians and clinicians. An effective amount of a peptide useful in the methods may be administered to a mammal in need thereof by any of a number of well-known methods for administering pharmaceutical compounds. The peptide may be administered systemically or locally.

The peptide may be formulated as a pharmaceutically acceptable salt. The term "pharmaceutically acceptable salt" means a salt prepared from a base or an acid which is acceptable for administration to a patient, such as a mammal (*e.g.*, salts having acceptable mammalian safety for a given dosage regime). However, it is understood that the salts are not required to be pharmaceutically acceptable salts, such as salts of intermediate compounds that are not intended for administration to a patient. Pharmaceutically acceptable salts can be derived from pharmaceutically acceptable inorganic or organic bases and from pharmaceutically acceptable inorganic or organic acids. In addition, when a peptide contains both a basic moiety, such as an amine, pyridine or imidazole, and an acidic moiety such as a carboxylic acid or tetrazole, zwitterions may be formed and are included within the term "salt" as used herein. Salts derived from pharmaceutically acceptable inorganic bases include ammonium, calcium, copper, ferric, ferrous, lithium, magnesium, manganic, manganous, potassium, sodium, and zinc salts, and the like. Salts derived from pharmaceutically acceptable organic bases include salts of primary, secondary and tertiary amines, including substituted amines, cyclic amines, naturally-occurring amines and the like, such as arginine, betaine, caffeine, choline, N,N'-dibenzylethylenediamine, diethylamine, 2-diethylaminoethanol, 2-dimethylaminoethanol, ethanolamine, ethylenediamine, N-ethylmorpholine, N-ethylpiperidine, glucamine, glucosamine, histidine, hydrabamine, isopropylamine, lysine, methylglucamine, morpholine, piperazine, piperadine, polyamine resins, procaine, purines, theobromine, triethylamine, trimethylamine, tripropylamine, tromethamine and the like. Salts derived from pharmaceutically acceptable inorganic acids include salts of boric, carbonic, hydrohalic (hydrobromic, hydrochloric, hydrofluoric or hydroiodic), nitric, phosphoric, sulfamic and sulfuric acids. Salts derived from pharmaceutically acceptable organic acids include salts of aliphatic hydroxyl acids *(e.g.,* citric, gluconic, glycolic, lactic, lactobionic, malic, and tartaric acids), aliphatic monocarboxylic acids (e.g., acetic, butyric, formic, propionic and trifluoroacetic acids), amino acids *(e.g.,* aspartic and glutamic acids), aromatic carboxylic acids *(e.g.,* benzoic, p-chlorobenzoic, diphenylacetic, gentisic, hippuric, and triphenylacetic acids), aromatic hydroxyl acids (e.g., o-hydroxybenzoic, p-hydroxybenzoic, 1-hydroxynaphthalene-2-carboxylic and 3-hydroxynaphthalene-2-carboxylic acids), ascorbic, dicarboxylic acids *(e.g.,* fumaric, maleic, oxalic and succinic acids), glucuronic, mandelic, mucic, nicotinic, orotic, pamoic, pantothenic, sulfonic acids *(e.g.*, benzenesulfonic, camphosulfonic, edisylic, ethanesulfonic, isethionic, methanesulfonic, naphthalenesulfonic, naphthalene-1,5-disulfonic, naphthalene-2,6-disulfonic and p-toluenesulfonic acids), xinafoic acid, and the like. In some embodiments, a pharmaceutically acceptable salt includes acetate salt or trifluoroacetate salt.

The aromatic-cationic peptides or a pharmaceutically acceptable salt thereof, such as acetate salt or trifluoroacetate salt, described herein can be incorporated into pharmaceutical compositions for administration, singly or in combination, to a subject for the treatment or prevention of a disorder described herein. Such compositions typically include the active agent and a pharmaceutically acceptable carrier. As used herein the term "pharmaceutically acceptable carrier" includes saline, solvents, dispersion media, coatings, antibacterial and antifungal agents, isotonic and absorption delaying agents, and the like, compatible with pharmaceutical administration. Supplementary active compounds can also be incorporated into the compositions.

Pharmaceutical compositions are typically formulated to be compatible with its intended route of administration. Examples of routes of administration include parenteral (*e.g.*, intravenous, intradermal, intraperitoneal or subcutaneous), oral, inhalation, transdermal (topical), intraocular, iontophoretic, and transmucosal administration. Solutions or suspensions used for parenteral, intradermal, or subcutaneous application can include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. pH can be adjusted with acids or bases, such as hydrochloric acid or sodium hydroxide. The parenteral preparation can be enclosed in ampoules, disposable syringes or multiple dose vials made of glass or plastic. For convenience of the patient or treating physician, the dosing formulation can be provided in a kit containing all necessary equipment (*e.g.*, vials of drug, vials of diluent, syringes and needles) for a treatment course *(e.g.,* 7 days of treatment).

Pharmaceutical compositions suitable for injectable use can include sterile aqueous solutions (where water soluble) or dispersions and sterile powders for the extemporaneous preparation of sterile injectable solutions or dispersion. For intravenous administration, suitable carriers include physiological saline, bacteriostatic water, Cremophor EL™ (BASF, Parsippany, N.J.) or phosphate buffered saline (PBS). In all cases, a composition for parenteral administration must be sterile and should be fluid to the extent that easy syringability exists. It should be stable under the conditions of manufacture and storage and must be preserved against the contaminating action of microorganisms such as bacteria and fungi.

The aromatic-cationic peptide compositions can include a carrier, which can be a solvent or dispersion medium containing, for example, water, ethanol, polyol (for example, glycerol, propylene glycol, and liquid polyethylene glycol, and the like), and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thiomerasol, and the like. Glutathione and other antioxidants can be included to prevent oxidation. In many cases, it will be preferable to include isotonic agents, for example, sugars, polyalcohols such as mannitol, sorbitol, or sodium chloride in the composition. Prolonged absorption of the injectable compositions can be brought about by including in the composition an agent which delays absorption, for example, aluminum monostearate or gelatin.

Sterile injectable solutions can be prepared by incorporating the active compound in the required amount in an appropriate solvent with one or a combination of ingredients enumerated above, as required, followed by filtered sterilization. Generally, dispersions are prepared by incorporating the active compound into a sterile vehicle, which contains a basic dispersion medium and the required other ingredients from those enumerated above. In the case of sterile powders for the preparation of sterile injectable solutions, typical methods of preparation include vacuum drying and freeze drying, which can yield a powder of the active ingredient plus any additional desired ingredient from a previously sterile-filtered solution thereof.

Oral compositions generally include an inert diluent or an edible carrier. For the purpose of oral therapeutic administration, the active compound can be incorporated with excipients and used in the form of tablets, troches, or capsules, *e.g*., gelatin capsules. Oral compositions can also be prepared using a fluid carrier for use as a mouthwash. Pharmaceutically compatible binding agents, and/or adjuvant materials can be included as part of the composition. The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate or Sterotes; a glidant such as colloidal silicon dioxide; a sweetening agent such as sucrose or saccharin; or a flavoring agent such as peppermint, methyl salicylate, or orange flavoring.

For administration by inhalation, the compounds can be delivered in the form of an aerosol spray from a pressurized container or dispenser which contains a suitable propellant, *e.g.,* a gas such as carbon dioxide, or a nebulizer.

Systemic administration of a therapeutic compound as described herein can also be by transmucosal or transdermal means. For transmucosal or transdermal administration, penetrants appropriate to the barrier to be permeated are used in the formulation. Such penetrants are generally known in the art, and include, for example, for transmucosal administration, detergents, bile salts, and fusidic acid derivatives. Transmucosal administration can be accomplished through the use of nasal sprays. For transdermal administration, the active compounds are formulated into ointments, salves, gels, or creams as generally known in the art. In one embodiment, transdermal administration may be performed my iontophoresis.

A therapeutic protein or peptide or a pharmaceutically acceptable salt thereof, such as acetate salt or trifluoroacetate salt can be formulated in a carrier system. The carrier can be a colloidal system. The colloidal system can be a liposome, a phospholipid bilayer vehicle. In one embodiment, the therapeutic peptide is encapsulated in a liposome while maintaining peptide integrity. As one skilled in the art would appreciate, there are a variety of methods to prepare liposomes. *(See* Lichtenberg et al., Methods Biochem. Anal., 33:337-462 (1988); Anselem et al., Liposome Technology, CRC Press (1993)). Liposomal formulations can delay clearance and increase cellular uptake *(See* Reddy, Ann. Pharmacother., 34(7-8):915-923 (2000)). An active agent can also be loaded into a particle prepared from pharmaceutically acceptable ingredients including, but not limited to, soluble, insoluble, permeable, impermeable, biodegradable or gastroretentive polymers or liposomes. Such particles include, but are not limited to, nanoparticles, biodegradable nanoparticles, microparticles, biodegradable microparticles, nanospheres, biodegradable nanospheres, microspheres, biodegradable microspheres, capsules, emulsions, liposomes, micelles and viral vector systems.

The carrier can also be a polymer, *e.g.,* a biodegradable, biocompatible polymer matrix. In one embodiment, the therapeutic peptide can be embedded in the polymer matrix, while maintaining protein integrity. The polymer may be natural, such as polypeptides, proteins or polysaccharides, or synthetic, such as poly α-hydroxy acids. Examples include carriers made of, *e.g.,* collagen, fibronectin, elastin, cellulose acetate, cellulose nitrate, polysaccharide, fibrin, gelatin, and combinations thereof. In one embodiment, the polymer is poly-lactic acid (PLA) or copoly lactic/glycolic acid (PGLA). The polymeric matrices can be prepared and isolated in a variety of forms and sizes, including microspheres and nanospheres. Polymer formulations can lead to prolonged duration of therapeutic effect. *(See* Reddy, Ann. Pharmacother., 34(7-8):915-923 (2000)). A polymer formulation for human growth hormone (hGH) has been used in clinical trials. *(See* Kozarich and Rich, Chemical Biology, 2:548-552 (1998)).

Examples of polymer microsphere sustained release formulations are described in PCT publication WO 99/15154 (Tracy et al.), U.S. Pat. Nos. 5,674,534 and 5,716,644 (both to Zale et al.), PCT publication WO 96/40073 (Zale et al.), and PCT publication WO 00/38651 (Shah et al.). U.S. Pat. Nos. 5,674,534 and 5,716,644 and PCT publication WO 96/40073 describe a polymeric matrix containing particles of erythropoietin that are stabilized against aggregation with a salt.

In some embodiments, the therapeutic compounds are prepared with carriers that will protect the therapeutic compounds against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Such formulations can be prepared using known techniques. The materials can also be obtained commercially, e.g., from Alza Corporation and Nova Pharmaceuticals, Inc. Liposomal suspensions (including liposomes targeted to specific cells with monoclonal antibodies to cell-specific antigens) can also be used as pharmaceutically acceptable carriers. These can be prepared according to methods known to those skilled in the art, for example, as described in U.S. Pat. No. 4,522,811.

The therapeutic compounds can also be formulated to enhance intracellular delivery. For example, liposomal delivery systems are known in the art, see, *e.g.,* Chonn and Cullis, "Recent Advances in Liposome Drug Delivery Systems," Current Opinion in Biotechnology 6:698-708 (1995); Weiner, "Liposomes for Protein Delivery: Selecting Manufacture and Development Processes," Immunomethods, 4(3):201-9 (1994); and Gregoriadis, "Engineering Liposomes for Drug Delivery: Progress and Problems," Trends Biotechnol., 13(12):527-37 (1995). Mizguchi et al., Cancer Lett., 100:63-69 (1996), describes the use of fusogenic liposomes to deliver a protein to cells both *in vivo* and *in vitro.*

Dosage, toxicity and therapeutic efficacy of the therapeutic agents can be determined by standard pharmaceutical procedures in cell cultures or experimental animals, *e.g.,* for determining the LD50 (the dose lethal to 50% of the population) and the ED50 (the dose therapeutically effective in 50% of the population). The dose ratio between toxic and therapeutic effects is the therapeutic index and it can be expressed as the ratio LD50/ED50. Compounds which exhibit high therapeutic indices are preferred. While compounds that exhibit toxic side effects may be used, care should be taken to design a delivery system that targets such compounds to the site of affected tissue in order to minimize potential damage to uninfected cells and, thereby, reduce side effects.

The data obtained from the cell culture assays and animal studies can be used in formulating a range of dosage for use in humans. The dosage of such compounds lies preferably within a range of circulating concentrations that include the ED50 with little or no toxicity. The dosage may vary within this range depending upon the dosage form employed and the route of administration utilized. For any compound used in the methods, the therapeutically effective dose can be estimated initially from cell culture assays. A dose can be formulated in animal models to achieve a circulating plasma concentration range that includes the IC50 *(i.e.,* the concentration of the test compound which achieves a half-maximal inhibition of symptoms) as determined in cell culture. Such information can be used to more accurately determine useful doses in humans. Levels in plasma may be measured, for example, by high performance liquid chromatography.

Typically, an effective amount of the aromatic-cationic peptides or a pharmaceutically acceptable salt thereof, such as acetate salt or trifluoroacetate salt, *e.g.,* SS-31 or a pharmaceutically acceptable salt thereof, such as acetate salt or trifluoroacetate salt, sufficient for achieving a therapeutic or prophylactic effect, range from about 0.000001 mg per kilogram body weight per day to about 10,000 mg per kilogram body weight per day. Suitably, the dosage ranges are from about 0.0001 mg per kilogram body weight per day to about 100 mg per kilogram body weight per day. For example dosages can be 1 mg/kg body weight or 10 mg/kg body weight every day, every two days, or every three days or within the range of 1-10 mg/kg every week, every two weeks or every three weeks. In one embodiment, a single dosage of peptide ranges from 0.001-10,000 micrograms per kg body weight. In one embodiment, aromatic-cationic peptide concentrations in a carrier range from 0.2 to 2000 micrograms per delivered milliliter. An exemplary treatment regime entails administration once per day or once a week. In therapeutic applications, a relatively high dosage at relatively short intervals is sometimes required until progression of the disease is reduced or terminated, and preferably until the subject shows partial or complete amelioration of symptoms of disease. Thereafter, the patient can be administered a prophylactic regime.

By way of example, and not by way of limitation, in one embodiment for the prevention or amelioration of MV-induced diaphragm weakness, an initial dose of cationic peptide (e.g., SS-31 or a pharmaceutically acceptable salt thereof, such as acetate salt or trifluoroacetate salt) is administered at about 1-20 mg/kg, about 1-15 mg/kg, about 1-10 mg/kg, about 1-5 mg/kg, 2-15 mg/kg, about 2-10 mg/k, about 2-5 mg/kg, about 2-3 mg/kg, or about 3 mg/kg. The initial dose is administered prior to, or shortly after MV begins. Additionally or alternatively, the initial dose is followed by a dose of about 0.01 mg/kg per hour, about 0.02 mg/kg per hour, about 0.03 mg/kg per hour, about 0.04 mg/kg per hour, about 0.05 mg/kg per hour, about 0.06 mg/kg per hour, about 0.07 mg/kg per hour, about 0.08 mg/kg per hour, about 0.09 mg/kg per hour, about 0.1 mg/kg per hour, about 0.2 mg/kg per hour, about 0.3 mg/kg per hour, about 0.5 mg/kg per hour, about 0.75 mg/kg per hour or about 1.0 mg/kg per hour.

In some embodiments, a therapeutically effective amount of an aromatic-cationic peptide or a pharmaceutically acceptable salt thereof, such as acetate salt or trifluoroacetate salt may be defined as a concentration of peptide at the target tissue of 10⁻¹² to 10⁻⁶ molar, e.g., approximately 10⁻⁷ molar. This concentration may be delivered by systemic doses of 0.001 to 100 mg/kg or equivalent dose by body surface area. The schedule of doses would be optimized to maintain the therapeutic concentration at the target tissue, most preferably by single daily or weekly administration, but also including continuous administration (e.g., parenteral infusion or transdermal application).

The skilled artisan will appreciate that certain factors may influence the dosage and timing required to effectively treat a subject, including but not limited to, the severity of the disease or disorder, previous treatments, the general health and/or age of the subject, and other diseases present. Moreover, treatment of a subject with a therapeutically effective amount of the therapeutic compositions described herein can include a single treatment or a series of treatments.

The mammal treated in accordance present methods can be any mammal, including, for example, farm animals, such as sheep, pigs, cows, and horses; pet animals, such as dogs and cats; laboratory animals, such as rats, mice and rabbits. In one embodiment, the mammal is a human.

In one embodiment, an additional therapeutic agent is administered to a subject in combination with an aromatic cationic peptide or a pharmaceutically acceptable salt thereof, such as acetate salt or trifluoroacetate salt, such that a synergistic therapeutic effect is produced. A "synergistic therapeutic effect" refers to a greater-than-additive therapeutic effect which is produced by a combination of two therapeutic agents, and which exceeds that which would otherwise result from individual administration of either therapeutic agent alone. Therefore, lower doses of one or both of the therapeutic agents may be used in treating muscle infirmities, resulting in increased therapeutic efficacy and decreased side-effects.

The multiple therapeutic agents may be administered in any order, simultaneously, sequentially or overlapping. If simultaneously, the multiple therapeutic agents may be provided in a single, unified form, or in multiple forms (by way of example only, either as a single pill or as two separate pills). One of the therapeutic agents may be given in multiple doses, or both may be given as multiple doses. If not simultaneous, the timing between the multiple doses may vary from more than zero weeks to less than four weeks. In addition, the combination methods, compositions and formulations are not to be limited to the use of only two agents.

### EXAMPLES

The present invention is further illustrated by the following examples, which should not be construed as limiting in any way.

### I. Example 1.

### A. Experimental Design

The purpose of this experiment was to demonstrate the role that mitochondrial ROS emission plays in MV-induced diaphragmatic weakness, and to demonstrate the effect of a mitochondrial-targeted antioxidant peptide (SS-31) on mitochondrial function and diaphragm muscle in rats. Two different groups of rats (1 and 2) were treated as follows.

### 1. Awake and spontaneously breathing rats

To determine the effect of a mitochondrial-targeted antioxidant (SS-31) on diaphragmatic contractile function, fiber cross sectional area (CSA), and mitochondrial function in awake and spontaneously breathing rats, animals were treated as follows. Animals (n=6/group) were randomly assigned into one of two experimental groups: (1) Control group-injected with saline (i.p.) at three hour intervals for 12 hours; and (2) Mitochondrial antioxidant group-injected (i.p.) with SS-31 every three hours for 12 hours. At the completion of the 12-hour treatment periods, diaphragmatic contractile function, fiber CSA, mitochondrial ROS emission, and mitochondrial respiratory function were measured.

The mitochondrial-targeted antioxidant SS-31 was dissolved in saline and delivered via four subcutaneous injections during the 12-hour experimental period. The first bolus (loading) dose (3 mg/kg; subcutaneous injection) was administered at the onset of the experiment. SS-31 (0.05 mg/kg/hr) was then administered via subcutaneous injections staged every three hours during the 12-hour experiment. All animals received the same total amount of SS-31 during 12 hours for all experiments requiring SS-31 administration.

### 2. Anesthetized rats

To analyze mitochondrial ROS emissions following MV-induced diaphragmatic oxidative stress and weakness, rats were randomly assigned to one of three experimental groups (n =12/group): (1) an acutely anesthetized control group; (2) a 12-hour MV group (MV); and 3) a 12-hour MV group treated with the mitochondrial-targeted antioxidant SS-31 (MVSS). Because of the large tissue requirement for our numerous dependent measures, six animals from each experimental group were used for the mitochondrial measures and the remaining six animals in each group were employed in all other biochemical assays.

Animals in the control group were acutely anesthetized with an intraperitoneal (IP) injection of sodium pentobarbital (60 mg/kg body weight). After reaching a surgical plane of anesthesia, the diaphragms were quickly removed. In one group of animals (n=6), a strip of the medial costal diaphragm was immediately used for in vitro contractile measurements, a separate section was stored for histological measurements, and the remaining portions of the costal diaphragm were rapidly frozen in liquid nitrogen and stored at -80°C for subsequent biochemical analyses. In a second group of animals (n=6), the entire costal diaphragm was rapidly removed and used to isolate mitochondria for measurements of mitochondrial respiration and ROS emission. The mitochondrial-targeted antioxidant SS-31 was dissolved in saline and delivered in a bolus (loading) dose (3 mg/kg; subcutaneous injection) 15 min prior to initiation of MV. A constant intravenous infusion (0.05 mg/kg/hr) of SS-31 was maintained throughout MV.

### B. Materials and methods:

*Mitochondrial-targeted antioxidant - Chemical details and Experimental delivery.* A mitochondria-targeted antioxidant designated as "SS-31" was selected for use in the current experiments. This molecule belongs to a family of small, water soluble peptides that contain an alternating aromatic-cationic motif and selectively target to the mitochondria. *See, e.g.,* Zhao et al., Cell-permeable peptide antioxidants targeted to inner mitochondrial membrane inhibit mitochondrial swelling, oxidative cell death, and reperfusion injury. The Journal of biological chemistry. Vol., 279(33): 34682-34690 (2004).

*Mechanical ventilation.* All surgical procedures were performed using aseptic techniques. Animals in the MV groups were anesthetized with an IP injection of sodium pentobarbital (60 mg/kg body weight), tracheostomized, and mechanically ventilated with a pressure-controlled ventilator (Servo Ventilator 300, Siemens) for 12 hours with the following settings: upper airway pressure limit: 20 cm H₂O, typical pressure generation above PEEP was 6-9 cm H₂O, respiratory rate: 80 bpm; and PEEP: 1 cm H₂O.

The carotid artery was cannulated to permit the continuous measurement of blood pressure and the collection of blood during the protocol. Arterial blood samples (100 µl per sample) were removed periodically and analyzed for arterial pO₂, pCO₂ and pH using an electronic blood-gas analyzer (GEM Premier 3000; Instrumentation Laboratory, Lexington, MA). Ventilator adjustments were made if arterial PCO₂ exceeded 40 mm Hg. Arterial PO₂ was maintained > 60 mmHg throughout the experiment by increasing the FIO₂ (22-26% oxygen).

A venous catheter was inserted into the jugular vein for continuous infusion of sodium pentobarbital (∼10 mg/kg/hr) and fluid replacement. Body temperature was maintained at 37°C by use of a recirculating heating blanket and heart rate was monitored via a lead II electrocardiograph. Continuous care during the MV protocol included lubricating the eyes, expressing the bladder, removing airway mucus, rotating the animal, and passively moving the limbs. Animals also received an intramuscular injection of glycopyrrolate (0.18 mg/kg) every two hours during MV to reduce airway secretions. Upon completion of MV, in one group of six animals the diaphragm was quickly removed and a strip of the medial costal diaphragm was used for in vitro contractile measurements, a section was stored for histochemical analyses, and the remaining portion was frozen in liquid nitrogen and stored at -80°C for subsequent analyses. In an additional group of animals (n=6), the entire costal diaphragm was rapidly removed and used to isolate mitochondria for measurements of mitochondrial respiration and ROS emission.

*Biochemical Measures.* Isolation of mitochondria. Approximately 500 mg of costal diaphragm muscle was used to isolate diaphragmatic mitochondria using the methods of Makinen and Lee (Makinen and Lee, Biochemical studies of skeletal muscle mitochondria. I. Microanalysis of cytochrome content, oxidative and phosphorylative activities of mammalian skeletal muscle mitochondria. Archives of biochemistry and biophysics., Vol., 126(1):75-82 (1968), with minor modifications. *See, e.g.,* Kavazis et al., Mechanical ventilation induces diaphragmatic mitochondrial dysfunction and increased oxidant production. Free radical biology & medicine., Vol., 46(6):842-850 (2009).

*Mitochondrial respiration.* Mitochondrial oxygen consumption was measured using previously described techniques. *See, e.g.,* Kavazis et al., Mechanical ventilation induces diaphragmatic mitochondrial dysfunction and increased oxidant production. Free radical biology & medicine. Vol., 46(6): 842-850 (2009). The maximal respiration (state 3) and state 4 respiration (basal respiration) were measured as described in Eastbrook et al. Mitochondrial respiratory control and the polarographic measurement of ADP/O ratios. Methods Enzymology. Vol., 10: 41-47 (1967). The respiratory control ratio (RCR) was calculated by dividing state 3 by state 4 respiration.

*Mitochondrial ROS emission.* Diaphragmatic mitochondrial ROS emission was determined using AmplexTM Red (Molecular Probes, Eugene, OR). Details of this assay have been described previously. *See, e.g.,* Kavazis *et al.* (2009). Mitochondrial ROS production was measured using the creatine kinase energy clamp technique to maintain respiration at steady state. Methodological details of this procedure have been described previously by Messer and collaborators. *See* Messer et al., Pyruvate and citric acid cycle carbon requirements in isolated skeletal muscle mitochondria. American journal of physiology. Vol., 286(3):C565-572 (2004). Finally, the rate of H₂O₂ emission was normalized to mitochondrial protein content.

*Western blot analysis.* Protein abundance was determined in diaphragm samples via Western Blot analysis using previously described methods. *See* McClung et al., Caspase-3 regulation of diaphragm myonuclear domain during mechanical ventilation-induced atrophy. Am J Respir Crit Care Med Vol., 175(2):150-159 (2007). After electrophoresis, the proteins were transferred to nitrocellulose membranes and incubated with primary antibodies directed against the protein of interest. 4-hydroxynonenal (4-HNE) (Abcam) was probed as a measurement indicative of oxidative stress while proteolytic activity was assessed by analyzing murf1 (ECM Biosciences), atrogin1 (ECM Biosciences), cleaved (active) calpain-1 (Cell Signaling) and cleaved (active) caspase-3 (Cell Signaling). Further, α-II spectrin (Santa Cruz) calpain specific cleavage (145 kDa cleavage product) and caspase-3 specific cleavage (120kDa cleavage product) were measured to obtain an additional measurement of both calpain-1 and caspase-3 activity during MV. The protein abundance of actin (Santa Cruz) was measured as an index of overall proteolysis in the diaphragm. Note that all membranes were stained with Ponceau S and analyzed to verify equal protein loading and transfer.

*Assessment of protein oxidation via reactive carbonyl* derivatives. The levels of reactive carbonyl derivatives in the myofibrillar protein samples were assessed as an index of the magnitude of protein modification. This was accomplished using the Oxyblot Oxidized Protein Detection Kit from Chemicon International (Temecula, Ca) as described previously. *See* Kavazis *et al.* (2009).

*RNA isolation and cDNA synthesis.* Total RNA was isolated from muscle tissue with TRIzol Reagent (Life Technologies, Carlsbad, CA) according to the manufacturer's instructions. RNA content (µg/mg muscle) was evaluated by spectrophotometry. RNA (5 µg) was then reverse transcribed with the Superscript III First-Strand Synthesis System for RT-PCR (Life Technologies), using oligo(dT)20 primers and the protocol outlined by the manufacturer.

*Real-time polymerase chain reaction.* One µl of cDNA was added to a 25 µl PCR reaction for real-time PCR using Taqman chemistry and the ABI Prism 7000 Sequence Detection system (ABI, Foster City, CA). Relative quantification of gene expression was performed using the comparative computed tomography method (ABI, User Bulletin #2). β-Glucuronidase, a lysosomal glycoside hydrolase, was chosen as the reference gene based on previous work showing unchanged expression with our experimental manipulations. *See, e.g.,* Deruisseau et al., Diaphragm Unloading via Controlled Mechanical Ventilation Alters the Gene Expression Profile. Am J Respir Crit Care Med. Vol., 172(10):1267-1275 (2005). MAFbx (GenBank NM AY059628) and MuRF-1 (GenBank NM AY059627, NM BC061824) mRNA transcripts were assayed using predesigned rat primer and probe sequences commercially available from Applied Biosystems (Assays-on-Demand).

*20S proteasome activity.* A section of the ventral costal diaphragm was homogenized and the in vitro chymotrypsin-like activity of the 20S proteasome was measured fluorometrically using techniques described by Stein and co-workers. *See* Stein et al., Kinetic characterization of the chymotryptic activity of the 20S proteasome. Biochemistry 35(13): 3899-3908 (1996).

*Functional Measures.* Measurement of in vitro diaphragmatic contractile properties. At the completion of the experimental periods, the entire diaphragm was removed and placed in a dissecting chamber containing a Krebs-Hensleit solution equilibrated with 95% O₂-5% CO₂ gas. A muscle strip (∼3mm wide), including the tendinous attachments at the central tendon and rib cage was dissected from the midcostal region. The strip was suspended vertically between two lightweight Plexiglas clamps with one end connected to an isometric force transducer (model FT-03, Grass Instruments, Quincy, MA) within a jacketed tissue bath. The muscle was electrically stimulated to contract and the force output was recorded *via* a computerized data-acquisition system as previously described. *See* Powers et al., Mechanical ventilation results in progressive contractile dysfunction in the diaphragm. J Appl Physiol, Vol. 92(5):1851-1858 (2002). For comparative purposes, diaphragmatic (bundles of fibers) force production was normalized as fiber cross sectional area (*i.e*., specific force production).

*Histological Measures.* Myofiber cross-sectional area. Sections from frozen diaphragm samples were cut at 10 microns using a cryotome (Shandon Inc., Pittsburgh, PA) and stained for dystrophin, myosin heavy chain (MHC) I and MHC type IIa proteins for fiber cross-sectional area analysis (CSA) as described previously. *See* McClung et al., Antioxidant administration attenuates mechanical ventilation-induced rat diaphragm muscle atrophy independent of protein kinase B (PKB Akt) signalling. J Physiol., Vol. 585:203-215 (2007). CSA was determined using Scion software (NIH).

*Statistical Analysis.* Comparisons between groups for each dependent variable were made by a one-way analysis of variance (ANOVA) and, when appropriate, a Tukey HSD (honestly significant difference) test was performed post-hoc. Significance was established at p < 0.05. Data are presented as means ± SEM.

*Measurement of mitochondrial protein carbonyl groups.* For mitochondrial protein extraction, ventricular tissues were homogenized in mitochondrial isolation buffer (1mM EGTA, 10 mM HEPES, 250 mM sucrose, 10 mM Tris-HCl, pH 7.4). The lysates were centrifuged for 7 min at 800g in 4°C. The supernatants were then centrifuged for 30 min at 4000g in 4 °C. The crude mitochondria pellets were resuspended in small volume of mitochondrial isolation buffer, sonicated on ice to disrupt the membrane, and treated with 1% streptomycin sulfate to precipitate mitochondrial nucleic acids. The OxiSelect™ Protein Carbonyl ELISA Kit (Cell Biolabs) was used to analyze 1 µg of protein sample per assay. The ELISA was performed according to the instruction manual, with slight modification. Briefly, protein samples were reacted with dinitrophenylhydrazine (DNPH) and probed with anti-DNPH antibody, followed by HRP conjugated secondary antibody. The anti-DNPH antibody and HRP conjugated secondary antibody concentrations were 1:2500 and 1:4000, respectively.

*Quantitative PCR.* Gene expression was quantified by quantitative real time PCR using an Applied Biosystems 7900 themocycler with Taqman Gene Expression Assays on Demand, which included: PGC1-α (Mm00731216), TFAM (Mm00447485), NRF-1 (Mm00447996), NRF-2 (Mm00487471), Collagen 1a2 (Mm00483937), and ANP (Mm01255747). Expression assays were normalized to 18S RNA.

*NADPH Oxidase activity.* The NADPH oxidase assay was performed as follows. In brief, 10 µg of ventricular protein extract was incubated with dihydroethidium (DHE, 10 µM), sperm DNA (1.25 µg/ml), and NADPH (50 µM) in PBS/DTPA (containing 100 µM DTPA), The assay was incubated at 37°C in the dark for 30 min and the fluorescence was detected using excitation/emission of 490/580 nm.

### C. Results:

### 1. SS-31 does not impact diaphragmatic fiber CSA or function in spontaneously breathing animals

To determine the impact of the mitochondrial antioxidant SS-31 on diaphragmatic contractile function, fiber cross sectional area (CSA), and mitochondrial function in awake and spontaneously breathing rats, animals were treated for 12-hours with the same levels of SS-31 that were provided to the mechanically ventilated animals during the 12-hour MV period. The results shown below in Tables 7A-7C demonstrate that, compared to untreated control animals, the treatment of animals with SS-31 does not influence diaphragmatic mitochondrial ROS emission and the mitochondrial respiratory ratio. Further, the results demonstrate that compared to control, treatment of animals with SS-31 did not alter diaphragmatic contractile function and fiber CSA.

| **Table 7A** | | |
|---|---|---|
| **Diaphragm muscle fiber type** | **Control Group** | **SS-31 Group** |
| | **Fiber CSA (µm²)** | **Fiber CSA (µm²)** |
| Type I | 1186±71 | 1280±44 |
| Type IIa | 1211±143 | 1267±49 |
| Type IIx/B | 3092±230 | 3007±304 |

Table **7A** shows fiber cross-sectional area (CSA) in diaphragm muscle fibers from both control (treated with saline injections) and awake and spontaneously breathing animals treated with the mitochondrial-targeted antioxidant SS-31. No significant differences in diaphragmatic fiber CSA existed between the Control and SS-31 groups in any fiber type. Values are means ± SEM.

| **Table 7B** | | |
|---|---|---|
| **Diaphragm stimulation frequency (Hz)** | **Control Group** | **SS-31 Group** |
| | **Diaphragm force production** (**Newtons**/**cm²**) | **Diaphragm force production** (**Newtons**/**cm²**) |
| 15 | **14**.**1±0**.**7** | **15**.**0±0**.**7** |
| 30 | **20**.**4±0**.**5** | **21**.**0±0**.**3** |
| 60 | 24.1±0.4 | **24**.**2±0**.**3** |
| 100 | **24**.**7±0**.**5** | **25.0±0.4** |
| 160 | **24**.**6±0**.**5** | **24**.**8±0**.**3** |

Table **7B** shows the effects of a mitochondrial targeted antioxidant (SS-31) on the diaphragmatic force-frequency response (*in vitro*) in control (saline injected) and SS-31 treated animals. No significant differences in diaphragmatic force production existed between the control and SS-31 groups at any stimulation frequency. Values are means ± SEM.

| **Table 7C** | | | | | | |
|---|---|---|---|---|---|---|
| **Group (N=4/ group)** | **H₂O₂ Emission State 3 (pmoles/min/mg)** | **H₂O₂ Emission State 4 (pmoles/min/mg)** | **State-3 VO₂** | **State-4 VO₂** | **ADP/O ratio** | **RCR** |
| **Control Group** | **51±3**.**6** | **661±21** | **282±28** | 67±5 | **2**.**2±0**.**2** | **4**.**3±0**.**3** |
| **SS-31 Group** | **54±4**.**5** | **652±18** | **237±15** | **49±2*** | **2**.**7±0**.**2** | **4**.**8±0**.**2** |

Table 7C shows the effects of a mitochondrial targeted antioxidant (SS-31) on diaphragm mitochondrial hydrogen peroxide emission and the mitochondrial respiratory function in control (saline injected) and SS-31 treated animals. These data were obtained using pyruvate/malate as substrate. VO₂ = mitochondrial oxygen consumption; RCR = respiratory control ratio. Units for state-3 and state-4 VO₂ are nmoles oxygen/mg protein/minute. Values are means ± SEM * = different from control at p<0.05.

### 2. Physiological responses to prolonged MV

To assess the efficacy of the MV protocol for maintaining homeostasis, arterial blood pressures, arterial PCO₂, arterial PO₂ and arterial pH were measured in all animals at the beginning of the experiments and at various time intervals during MV. Although small variations in arterial blood pressure, blood gases, and pH existed over time, our results confirm that arterial blood pressure and blood-gas/pH homeostasis were well-maintained during MV (Table 8).

| **Table 8** | | |
|---|---|---|
| **Physiological variable** | MV | MVSS |
| **Heart rate (beats/min)** | 339±10 | 347±7 |
| **Systolic blood pressure (mm/Hg)** | 105±6 | 108±5 |
| **Arterial PO₂ (mm/Hg)** | 73±2 | 75±5 |
| **Arterial PCO₂** | 45±0.8 | 46±1 |
| **Arterial pH** | 7.41 ±0.01 | 7.41 ±0.01 |

**Table 8** shows animal heart rates, systolic blood pressure, and arterial blood gas tension/pH and at the completion of 12 hours of mechanical ventilation. Values are means ± SEM. No significant differences existed between the two experimental groups in any of these physiological variables.

In addition, strict aseptic techniques were followed throughout the experiments given that sepsis is associated with diaphragmatic contractile dysfunction. Importantly, the data illustrate that animals did not develop infection during MV. This is supported by the observation that microscopic examination of blood revealed no detectable bacteria, and that postmortem (visual) examination of the lungs and peritoneal cavity yielded no detectable abnormalities. Furthermore, MV animals were afebrile during the investigation, with body temperatures ranging from 36.3 to 37.4°C. Finally, during the course of MV, no significant (P<0.05) changes occurred in the body weights of the MV animals. Collectively, these results indicate that the MV animals were significantly free of any infection.

As compared to controls, the results show that treatment of spontaneous breathing animals with SS-31 did not alter any of these dependent measures (see below). Therefore, further experiments were performed using SS-31 as a mitochondrial-targeted antioxidant to analyze mitochondrial ROS emissions during MV-induced diaphragmatic weakness, which consisted ofMV for 12-hours.

### 3. SS-31 impedes MV-induced ROS emission from diaphragmatic mitochondria

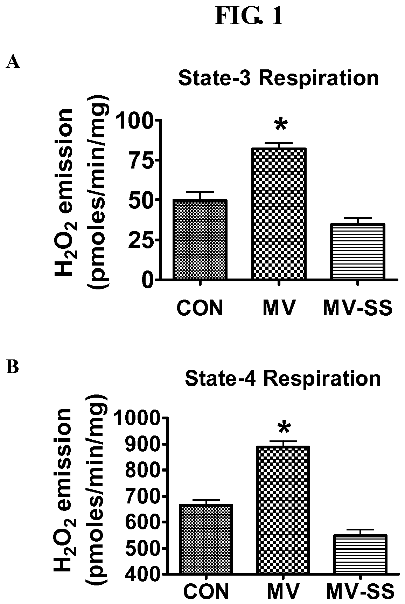

Mitochondrial-derived ROS emissions were assessed in mitochondria for an association with MV-induced oxidative damage, contractile dysfunction, and atrophy in the diaphragm. In this respect, rats were treated with a mitochondrial-targeted antioxidant (SS-31) to prevent MV-induced ROS emission from diaphragm mitochondria. It is noted that treatment with SS-31 prevented the MV-induced increase in diaphragmatic mitochondrial H₂O₂ release both during state 3 and 4 mitochondrial respiration. *See* **FIG. 1****.** In this regard, hydrogen peroxide release from mitochondria isolated from diaphragms of mechanically ventilated (MV) rats, in the absence of SS-31 did not show a decrease. As such, treatment of animals with SS-31 significantly reduced the rates of H₂O₂ release from the mitochondria following prolonged MV. Values are mean ± SEM. * = different (p<0.05) from both CON and MVSS (n =6/group). *See* **FIG. 1****.**

Prolonged MV results in damage to mitochondria as indicated by impaired coupling (*i.e*., lower respiratory control ratios) in mitochondria isolated from the diaphragm of MV animals. Therefore, treatment of animals with SS-31 protects diaphragmatic mitochondria from MV-induced mitochondrial uncoupling. As shown in Table 9, treatment with SS-31 was successful in averting diaphragmatic mitochondrial uncoupling that occurs following prolonged MV.

**Table 9**

| **Parameter** | **Control** | **MV** | **MVSS** |
|---|---|---|---|
| State-3 VO₂ | 235.9±10 | 212.4±11 | 193.1±9 |
| State-4 VO₂ | 61.8±3 | 77.6±5* | 42.4±3 |
| RCR | 4.7±0.2 | 2.7±0.3* | 4.6±0.2 |
| ADP/O ratio | 2.1±0.2 | 2.3±0.2 | 2.3±0.2 |

**Table 9** shows state-3 respiration, state-4 respiration, and respiratory control ratio (RCR) in mitochondria isolated from diaphragms of control (CON), mechanically ventilated (MV), and mechanically ventilated animals treated with the mitochondrial antioxidant, SS-31 (MVSS). These data were obtained using pyruvate/malate as substrate. Units for state-3 and state-4 oxygen consumption (VO₂) are nmoles oxygen/mg protein/minute. Values are means ± SEM. * different (p<0.05) from both CON and MVSS.

### 4. MV-induced oxidative stress is mediated by mitochondrial ROS emission

To determine if mitochondrial ROS emission is required for MV-induced oxidative stress in the diaphragm, two biomarkers of oxidative damage were measured, *i.e.,* diaphragmatic levels of 4-HNE-conjugated cytosolic proteins and levels of protein carbonyls in myofibrillar proteins. The results reveal that treatment of animals with SS-31 protected the diaphragm against the ROS-induced increase in both protein carbonyls and 4-HNE-conjugated proteins normally associated with prolonged MV. *See* **FIG. 2****.** In this respect, levels of oxidatively modified proteins in the diaphragm of control (CON), mechanically ventilated (MV), and mechanically ventilated rats treated with the mitochondrial-targeted antioxidant SS-31 (MVSS) were measured.

As shown in **FIG. 2A****,** levels of 4-hydroxyl-nonenal-conjugated proteins in the diaphragm of the three experimental groups are listed. The image above the histograph is a representative western blot of data from the three experimental groups. **FIG. 2B** further illustrates the levels of protein carbonyls in the diaphragm of the three experimental groups. * = different (p<0.05) from both CON and MVSS (n = 6/group). *See* **FIG. 2****.**

### 5. Increased mitochondrial ROS emission is required for MV-induced diaphragmatic contractile dysfunction and fiber atrophy

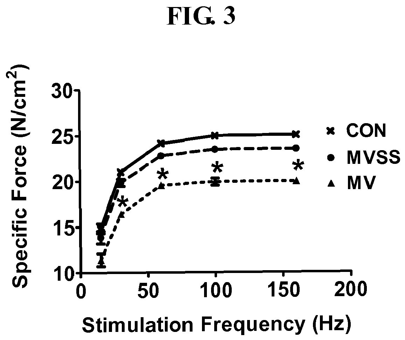

To assess the role that mitochondrial ROS emission plays in MV-induced diaphragmatic contractile dysfunction, diaphragmatic contractile performance *in vitro* using strips of diaphragm muscle obtained from control, MV, and MV animals treated with SS-31 were measured. Prevention of mitochondrial ROS emission using SS-31 successfully prevented the diaphragmatic contractile dysfunction associated with prolonged MV. *See* FIG. 3. As shown in **FIG. 3****,** prolonged MV effects the diaphragmatic force-frequency response (*in vitro*) in control and mechanically ventilated rats with/without mitochondrial targeted antioxidants. However, no significant differences in diaphragmatic force production existed between the CON and MVSS groups at any stimulation frequency. Values are means ± SEM. Note that some of the SEM bars are not visible because of the small size. * = different (p<0.05) from both CON and MVSS (n = 6/group). *See* **FIG. 3****.**

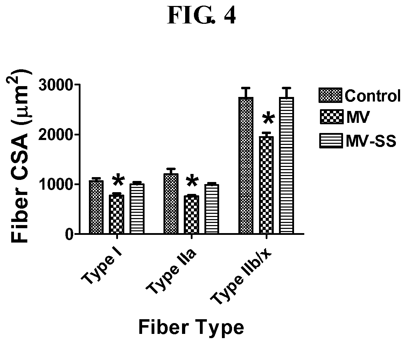

MV-induced oxidative stress is a requirement for the diaphragmatic fiber atrophy that is associated with prolonged MV. *See* Betters et al., Trolox attenuates mechanical ventilation-induced diaphragmatic dysfunction and proteolysis. Am J Respir Crit Care Med., Vol., 170(11):1179-1184 (2004). As shown in **FIG. 4****,** fiber cross-sectional area (CSA) in diaphragm muscle myofibers from control (CON) and mechanically ventilated rats with (MVSS) and without mitochondrial targeted antioxidants (MV) were tested. It is noted that no significant differences in diaphragmatic fiber CSA existed between the CON and MVSS groups in any fiber type. Values are means ± SEM. * = different (p<0.05) from both CON and MVSS (n = 6/group). *See* **FIG. 4****.** It was determined that MV-induced mitochondrial ROS emission is a requirement for MV-induced diaphragmatic atrophy. Myofiber cross-sectional area was determined for individual fiber types for all treatment groups. The data indicates that prevention of the MV-induced increase in mitochondrial ROS emission protects the diaphragm from MV-induced fiber atrophy. *See* **FIG. 4****.**

### 6. MV-induced mitochondrial ROS emission promotes diaphragmatic protease activation and proteolysis

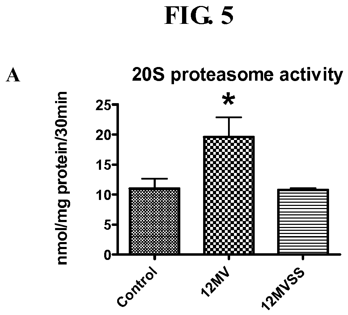

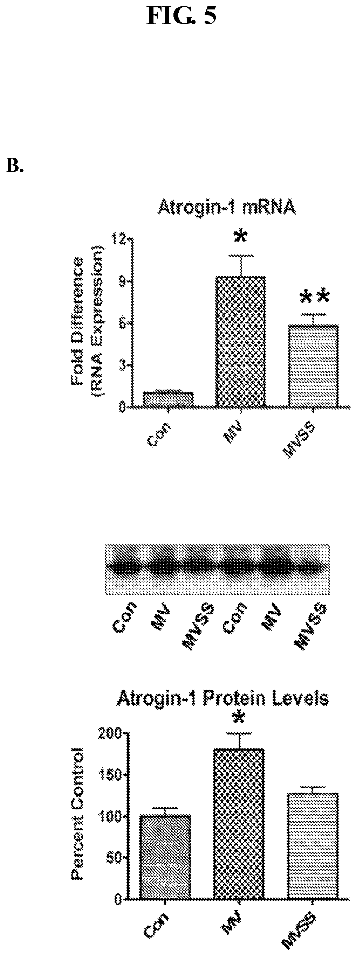

The ubiquitin-proteasome system of proteolysis is activated in the diaphragm during prolonged MV and therefore likely contributes to MV-induced diaphragmatic protein breakdown. To determine the effects of mitochondrial ROS emission on the ubiquitin-proteasome system of proteolysis, 20S proteasome activity was measured along with both mRNA and protein levels of two important muscle specific E3 ligases (*i.e*., atrogin-1/MAFbx and MuRF-1) in the diaphragm. The results reveal that prevention of MV-induced mitochondrial ROS release *via* SS-31 prevented the MV-induced increase in 20S proteasome activity in the diaphragm. *See* **FIG. 5A****.** Further, the results indicate that prolonged MV resulted in a significant increase in atrogin-1/MAFbx mRNA levels in the diaphragm of both MV groups; however, treatment of animals with SS-31 significantly blunted the MV-induced increase in atrogin-1/MAFbx protein levels in the diaphragm. *See* **FIG. 5B**.

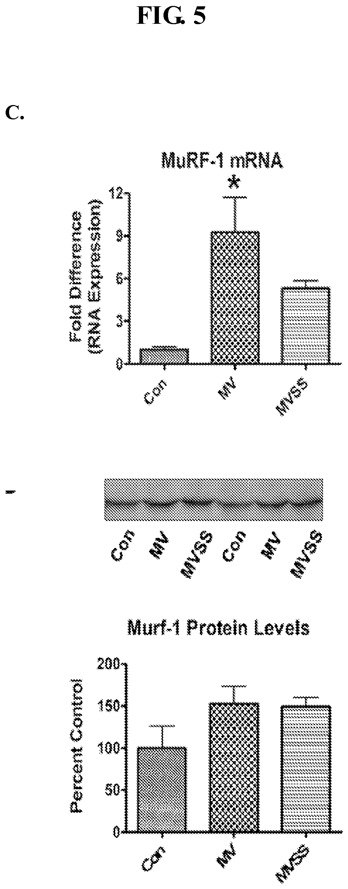

**FIG. 5C** illustrates the impact of prolonged MV on both diaphragmatic mRNA and protein levels of MuRF-1. Prolonged MV resulted in a significant increase in MuRF-1 mRNA levels in the diaphragm and although MuRF-1 proteins levels tended to increase in the diaphragm of mechanically ventilated animals, these differences did not reach significance. The images above the histograms in **FIG. 5B-C** are representative western blots of data from the three experimental groups. Values are means ± SEM. * = different (p<0.05) from both CON and MVSS. ** = different (p<0.05) from both CON and MV (n = 6/group). *See* **FIG. 5****.**

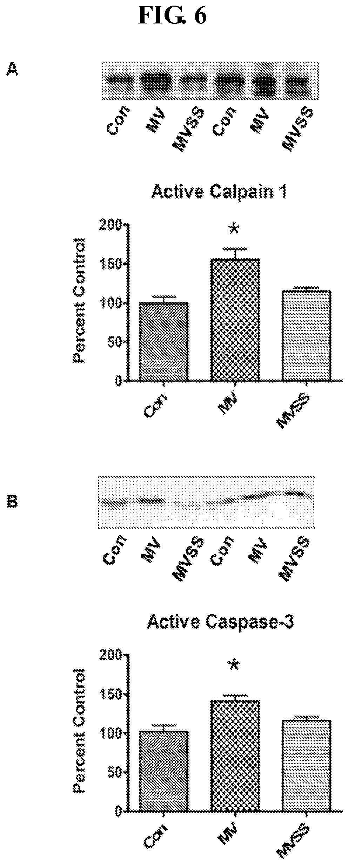

Calpain and caspase-3 activation in the diaphragm has an important role in MV-induced diaphragmatic atrophy and contractile dysfunction. *See* McClung et al., Caspase-3 regulation of diaphragm myonuclear domain during mechanical ventilation-induced atrophy, Am J Respir Crit Care Med., Vol. 175(2):150-159 (2007). Diaphramatic calpain and caspase-3 activity were assayed using two different but complimentary methods. First, active calpain-1 and caspase-3 levels in the muscle were determined *via* Western blot to detect the cleaved and active forms of calpain 1 and caspase-3. *See* **FIG. 6****.** As shown in **FIG. 6A****,** the active form of calpain 1 in diaphragm muscle is detected at the completion of 12 hours of MV. The cleaved and active band of caspase-3 in diaphragm muscle at the completion of 12 hours of MV is also illustrated. *See* **FIG. 6B****.** The images above the histograms in **FIGS. 6A** and **6B** are representative western blots of data from the three experimental groups. Values are means ± SEM. * = different (p<0.05) from both CON and MVSS (n=6/group). *See* **FIG. 6B****.**

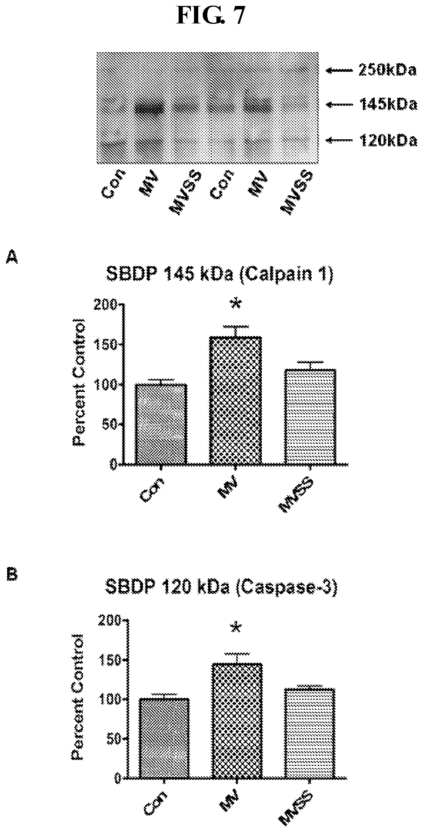

Calpain 1 and caspase-3 activity were measured at one time period. Therefore, calpain and caspase-3 specific degradation products of αII-spectrin were also measured as these breakdown products provide an *in vivo* signature that can be detected. *See* **FIG. 7****.** This technique provides an index of *in vivo* calpain and caspase-3 activity in the diaphragm over a prolonged period of time during MV. As shown in **FIG. 7A****,** levels of the 145 kDa α-II-spectrin degradation product (SBPD) in diaphragm muscle following 12 hours of MV are measured. It is noted that the SBDP 145 kDa is an α-II-spectrin degradation product specific to calpain cleavage of intact α-II-spectrin and therefore, the cellular level of SBDP 145 kDa is employed as a biomarker of *in vivo* calpain activity.

As shown in **FIG. 7B****,** levels of the 120 kDa α-II-spectrin break-down product (SBPD 120 kDa) in diaphragm muscle following 12 hours of MV were measured. It is noteworthy that the SBDP 120 kDa is a α-II-spectrin degradation product specific to caspase-3 cleavage of intact α-II-spectrin and therefore, the cellular levels of SBDP 120 kDa can be used as a biomarker of caspase-3 activity. The images above the FIGS. 7A and 7B histograms are representative western blots of data from the three experimental groups. Values are means ± SEM. * = different (p<0.05) from both CON and MVSS (n=6/group). *See* FIG. 7.

Together these results demonstrate that treatment of animals with a mitochondrial-targeted antioxidant (SS-31) protected the diaphragm against the activation of both calpain and caspase-3. *See* FIGS. 6-7. These findings illustrate that mitochondria are the dominant source of MV-induced ROS production in the diaphragm and that mitochondrial ROS production is essential for MV-induced activation of both calpain and caspase-3 in the diaphragm.

### 7. Mitochondrial-targeted antioxidants protect against MV-induced diaphragmatic proteolysis

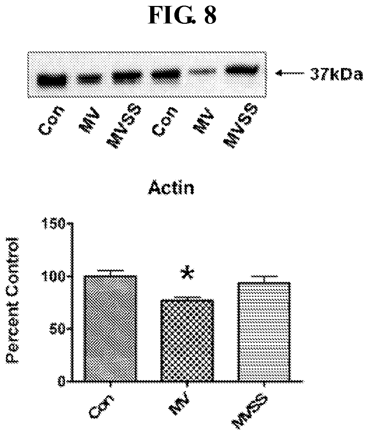

After demonstrating that prevention of MV-induced increases in mitochondrial ROS emission protects the diaphragm against protease activation, the relative abundance of the sarcomeric protein actin in the diaphragm as a marker of disuse-induced muscle proteolysis was measured. Since actin is preferentially degraded during disuse muscle atrophy, assessment of the actin protein levels provides an index of proteolysis. *See* Li et al., Interleukin-1 stimulates catabolism in C2C12 myotubes. American Journal of Physiology., Vol., 297(3):C706-714 (2009). The results reveal that, compared to diaphragm muscle from both control and MV-SS animals, the actin abundance was significantly reduced in diaphragm muscle from animals exposed to prolonged MV without mitochondrial antioxidants. *See* **FIG. 8****.** Therefore, prevention of MV-induced mitochondrial ROS emission not only protected against protease activation, this treatment protected against MV-induced diaphragmatic proteolysis.

As shown in **FIG. 8****,** the ratio of actin to total sarcomeric protein levels in the diaphragm from control (CON) and mechanically ventilated animals with (MVSS) without mitochondrial-targeted antioxidants (MV) was measured. Because actin is preferentially degraded during disuse muscle atrophy, assessment of the ratio of actin to total sarcomeric protein levels provides a relative index of diaphragmatic proteolysis during prolonged MV. The image above the histogram is a representative western blot of data from the three experimental groups. Values are means ± SEM. * = different (p<0.05) from both CON and MVSS (n=6/group). *See* **FIG. 8****.**

### II. Example 2

### A. Experimental Design and methods:

The purpose of this example was to demonstrate that MV-induced mitochondrial oxidation is generalizable to disuse-induced skeletal muscle weakness. Two different groups of mice (1 and 2) were treated as follows.

### 1. Normal, mobile mice

Normal, mobile mice were randomly divided into two groups, A and B, with 8 mice per group. Group A mice received an an injection of sterile saline; Group B mice received an injection of the mitochondrial targeted peptide SS-31.

### 2. Hindlimb casted mice

Mouse hind limbs were immobilized by casting for 14 days, thereby inducing hind limb muscle atrophy. Casted mice received an injection of sterile saline (0.3 ml) or an injection containing the peptide SS-31 (0.3 ml). A control group of untreated mice was also used in this experiement.

### B. Materials and methods:

### Animals

Seventy-two adult male C57B16 mice (age 21-28 weeks, body weight 26.44±0.54g) were used in these experiments. Animals were maintained on a 12:12 hour light-dark cycle and provided food (AIN93 diet) and water *ad libitum* throughout the experimental period. The Institutional Animal Care and Use Committee of the University of Florida approved these experiments.

### Experimental Design

To test the hypothesis that mitochondrial ROS production plays a role in immobilization-induced skeletal muscle atrophy, mice were randomly assigned to one of three experimental groups (n =24/group): 1) no treatment (Control) group; 2) 14 days of hind-limb immobilization group (Cast); and 3) 14 days of hind-limb immobilization group treated with the mitochondrial-targeted antioxidant SS-31 (Cast+SS). Note that 14-days of hind-limb immobilization group (Cast) received saline infusions whereas animals in the group were treated with the mitochondrial-targeted antioxidant SS-31 during immobilization period.

### Experimental Protocol

**Immobilization.** Mice were anesthetized with gaseous isoflurane (3% induction, 0.5-2.5% maintenance). Anesthetized animals were cast-immobilized bilaterally with the ankle joint in the plantar-flexed position to induce maximal atrophy of the soleus and plantaris muscle. Both hindlimbs and the caudal fourth of the body were encompassed by a plaster of paris cast. A thin layer of padding was placed underneath the cast in order to prevent abrasions. In addition, to prevent the animals from chewing on the cast, one strip of fiberglass material was applied over the plaster. The mice were monitored on a daily basis for chewed plaster, abrasions, venous occlusion, and problems with ambulation.

**Mitochondrial-targeted antioxidant administration.** Mice in the hind-limb immobilization group received daily subcutaneous injections of the mitochondrial-targeted antioxidant SS-31 dissolved in saline (1.5 mg/kg) during the immobilization period. SS-31 was chosen due to its specificity as a mitochondrial-targeted antioxidant (Zhao K, Zhao GM, Wu D, Soong Y, Birk AV, Schiller PW, Szeto HH. Cell-permeable peptide antioxidants targeted to inner mitochondrial membrane inhibit mitochondrial swelling, oxidative cell death, and reperfusion injury. The Journal of biological chemistry 2004;279:34682-34690).

### Biochemical Measures

**Preparation of permeabilized muscle fibers.** This technique has been adapted from previous methods (Korshunov SS, et al., High protonic potential actuates a mechanism of production of reactive oxygen species in mito- chondria. FEBS Lett 416: 15-18, 1997; Tonkonogi M, et al., Reduced oxidative power but unchanged antioxidative capacity in skeletal muscle from aged humans. Pflügers Arch 446: 261-269, 2003). Briefly, small portions (∼25 mg) of soleus and planatris muscle were dissected and placed on a plastic Petri dish containing ice-cold *buffer X* (60 mM K-MES, 35 mM KCl, 7.23 mM K2EGTA, 2.77 mM CaK2EGTA, 20 mM imidazole, 0.5 mM DTT, 20 mM taurine, 5.7 mM ATP, 15 mM PCr, and 6.56 mM MgCl2, pH 7.1). The muscle was trimmed of connective tissue and cut down to fiber bundles (4-8 mg wet wt). Under a microscope and using a pair of extra-sharp forceps, the muscle fibers were gently separated in ice-cold buffer X to maximize surface area of the fiber bundle. To permeabilize the myofibers, each fiber bundle was incubated in ice-cold buffer X containing 50µg/ml saponin on a rotator for 30 min at 4°C. The permeabilized bundles were then washed in ice-cold *buffer Z* (110 mM K-MES, 35 mM KCl, 1 mM EGTA, 5 mM K2HPO4, and 3 mM MgCl2, 0.005 mM glutamate, and 0.02 mM malate and 0.5 mg/ml BSA, pH 7.1)

**Mitochondrial respiration in permeabilized fibers.** Respiration was measured polarographically in a respiration chamber maintained at 37°C (Hansatech Instruments, United Kingdom). After the respiration chamber was calibrated, permeabilized fiber bundles were incubated with 1 ml of respiration buffer Z containing 20 mM creatine to saturate creatine kinase (Saks VA, et al. Permeabilized cell and skinned fiber techniques in studies of mitochondrial function in vivo. Mol Cell Biochem 184: 81-100, 1998; Walsh B, et al. The role of phosphorylcreatine and creatine in the regulation of mitochondrial respiration in human skeletal muscle. J Physiol 537: 971- 978, 2001). Flux through complex I was measured using 5 mM pyruvate and 2 mM malate. The maximal respiration (state 3), defined as the rate of respiration in the presence of ADP, was initiated by adding 0.25 mM ADP to the respiration chamber. Basal respiration (state 4) was determined in the presence of 10 µg/ml oligomycin to inhibit ATP synthesis. The respiratory control ratio (RCR) was calculated by dividing state 3 by state 4 respiration.

**Mitochondrial ROS production.** Mitochondrial ROS production was determined using Amplex™ Red (Molecular Probes, Eugene, OR). The assay was performed at 37°C in 96-well plates using succinate as the substrate. Specifically, this assay was developed on the concept that horseradish peroxidase catalyzes the H₂O₂-dependent oxidation of non-fluorescent Amplex™ Red to fluorescent Resorufin Red, and it is used to measure H₂O₂ as an indicator of superoxide production. Superoxide dismutase (SOD) was added at 40 units/ml to convert all superoxide into H₂O₂. We monitored Resorufin formation (Amplex™ Red oxidation by H₂O₂) at an excitation wavelength of 545 nm and an emission wavelength of 590 nm using a multiwell plate reader flurometer (SpectraMax, Molecular Devices, Sunnyvale, CA). The level of Resorufin formation was recorded every 5 minutes for 15 minutes, and H₂O₂ production was calculated with a standard curve.

**Western blot analysis.** Protein abundance was determined in skeletal samples via Western Blot analysis. Briefly, soleus and plataris tissue samples were homogenized 1:10 (wt/vol) in 5 mM Tris (pH 7.5) and 5 mM EDTA (pH 8.0) with a protease inhibitor cocktail (Sigma) and centrifuged at 1500 g for 10 min at 4°C. After collection of the resulting supernatant, muscle protein content was assessed by the method of Bradford (Sigma, St. Louis). Proteins were separated using electrophoresis via 4-20% polyacrylamide gels containing 0.1% sodium dodecyl sulfate for ∼1 h at 200 V. After electrophoresis, the proteins were transferred to nitrocellulose membranes and incubated with primary antibodies directed against the protein of interest. 4-HNE (Abcam) was probed as a measurement indicative of oxidative stress while proteolytic activity was assessed by cleaved (active) calpain-1 (Cell Signaling) and cleaved (active) caspase-3 (Cell Signaling). Following incubation, membranes were washed with PBS-Tween and treated with secondary antibody (Amersham Biosciences). A chemiluminescent system was used to detect labeled proteins (GE Healthcare) and membranes were developed using autoradiography film and a developer (Kodak). The resulting images were analyzed using computerized image analysis to determine percentage change from control. Membranes were stained with Ponceau S and analyzed to verify equal protein loading and transfer.

### Histological Measures

**Myofiber cross-sectional area.** Sections from frozen soleus and plantaris samples (supported in OCT) were cut at 10 microns using a cryotome (Shandon Inc., Pittsburgh, PA) and stained for dystrophin, myosin heavy chain (MHC) I and MHC type IIa proteins for fiber cross-sectional area analysis (CSA) as described previously (McClung JM, et al., Antioxidant administration attenuates mechanical ventilation-induced rat diaphragm muscle atrophy independent of protein kinase b (pkb akt) signalling. J Physiol 2007;585:203-215). CSA was determined using Scion software (NIH).

### Statistical Analysis

Comparisons between groups for each dependent variable were made by a one-way analysis of variance (ANOVA) and, when appropriate, a Tukey HSD (honestly significant difference) test was performed *post-hoc.* Significance was established at p < 0.05. Data are presented as means ± SEM.

### C. Results:

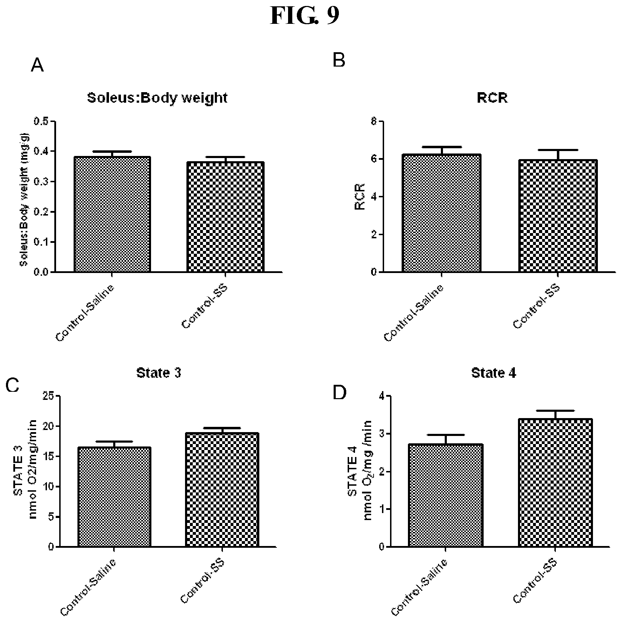

As shown in Figures **9-18****,** SS-31 had no effect on normal skeletal muscle size or mitochondrial function. However, SS-31 was able to prevent oxidative damage and associated muscle weakness (*e.g.,* atrophy, contractile dysfunction, etc.) emanating from hind limb immobilization.

### 1. Normal, mobile mice

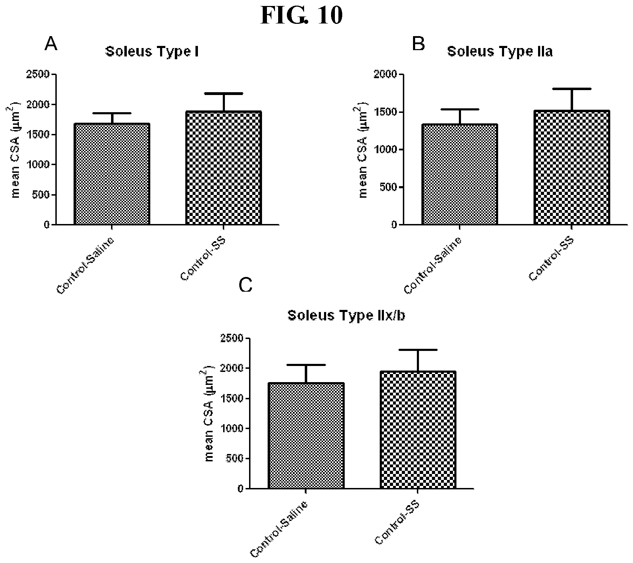

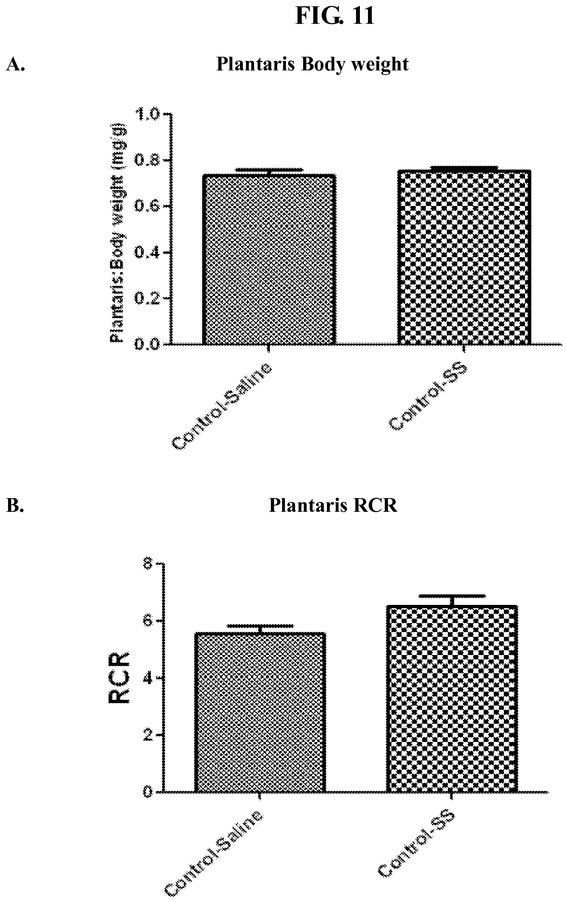

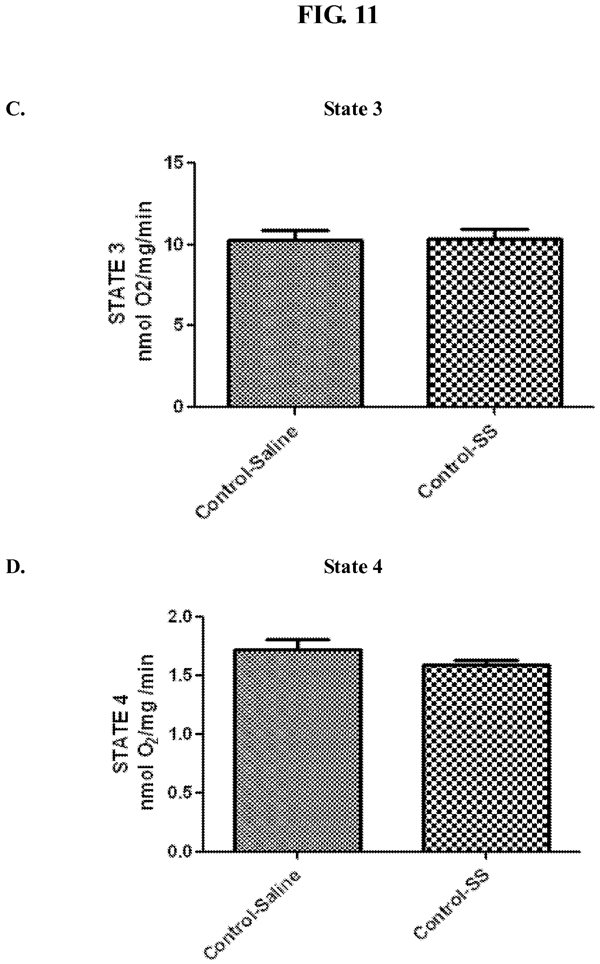

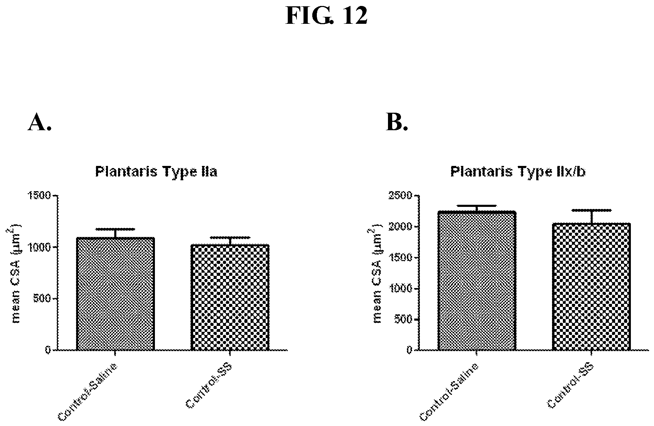

As illustrated by **FIG. 9A-D,** SS-31 had no effect on soleus muscle weight, the respiratory coupling ratio (RCR), mitochondrial state 3 respiration, or mitochondrial state 4 respiration, respectively in mobile mice. RCR is the respiratory quotient ratio of state 3 to state 4 respiration, as measured by oxygen consumption. Likewise, **FIG. 10A-C** show that SS-31 did not have any variable effects on muscle fibers of different size in normal soleus muscle. Furthermore, as illustrated by **FIG. 11A-D,** SS-31 had no effect on plantaris muscle weight, the respiratory coupling ratio (RCR), mitochondrial state 3 respiration, or mitochondrial state 4 respiration, respectively. Similarly, **FIG. 12A-B** shows that SS-31 did not impart any variable effects to the muscle fibers of different size in normal plantaris muscle fiber tissue.

### 2. Hindlimb casted mice

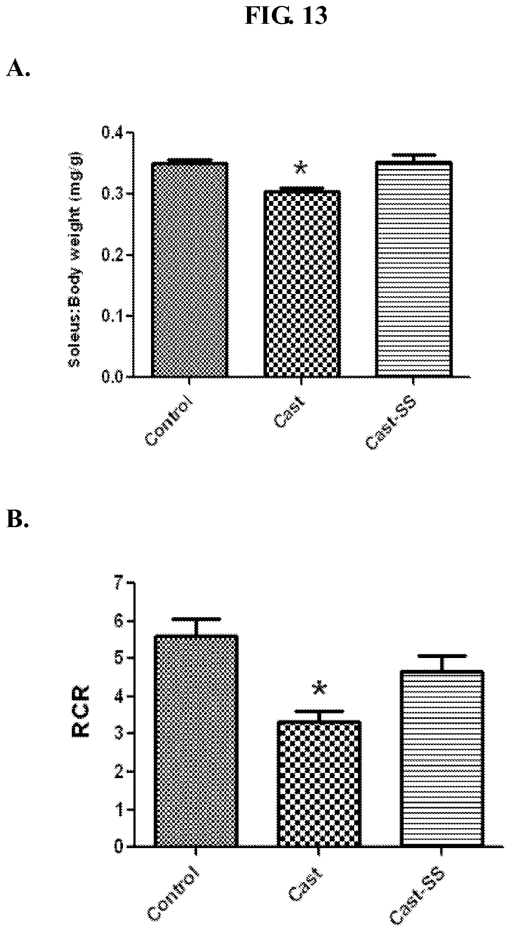

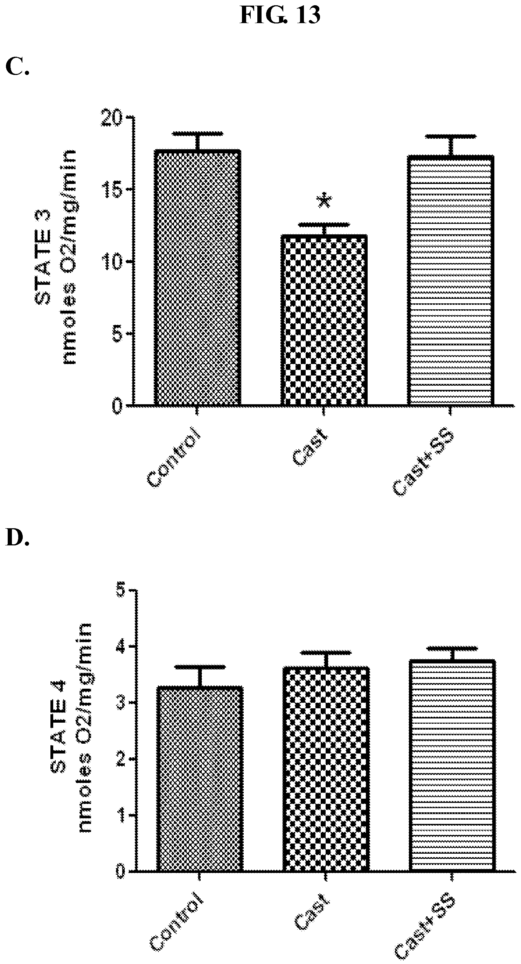

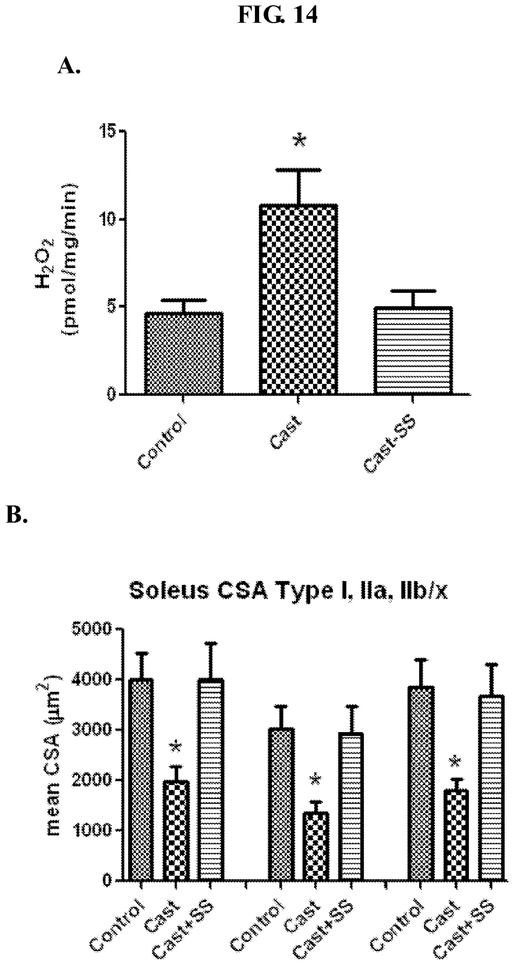

As shown by **FIG. 13A-D,** casting for 7 days led to a significant decrease in soleus muscle weight (**FIG**. **13A**), RCR **(****FIG. 13B****),** and mitochondrial state 3 respiration **(****FIG. 13C****),** all of which was reversed by administration of SS-31. The casting did not have a significant effect on state 4 respiration. Likewise, casting for 7 days significantly increased H₂O₂ production by mitochondria isolated from soleus muscle, which was similarly prevented by SS-31. *See* **FIG. 14A-B.** As shown in **FIG. 14B****,** SS-31 prevented cross sectional area loss for three types of fibers in the soleus (type I, IIa and IIb/x).

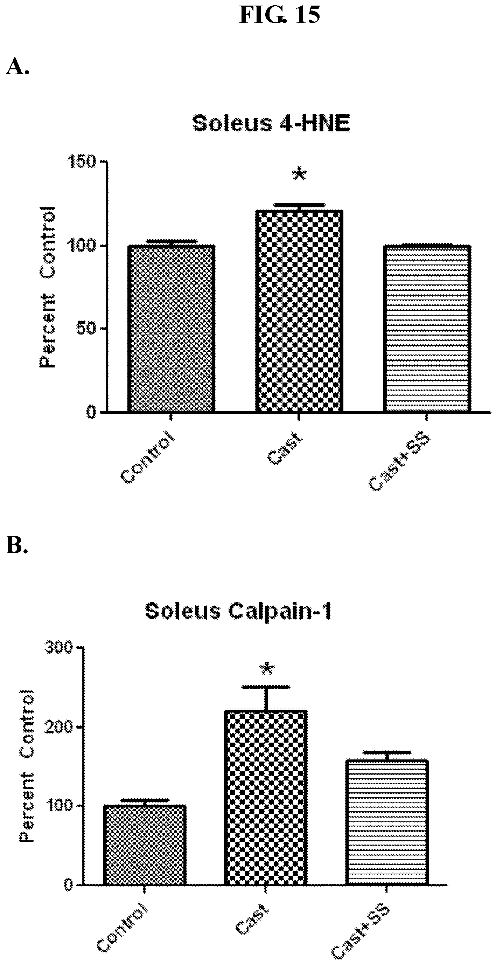

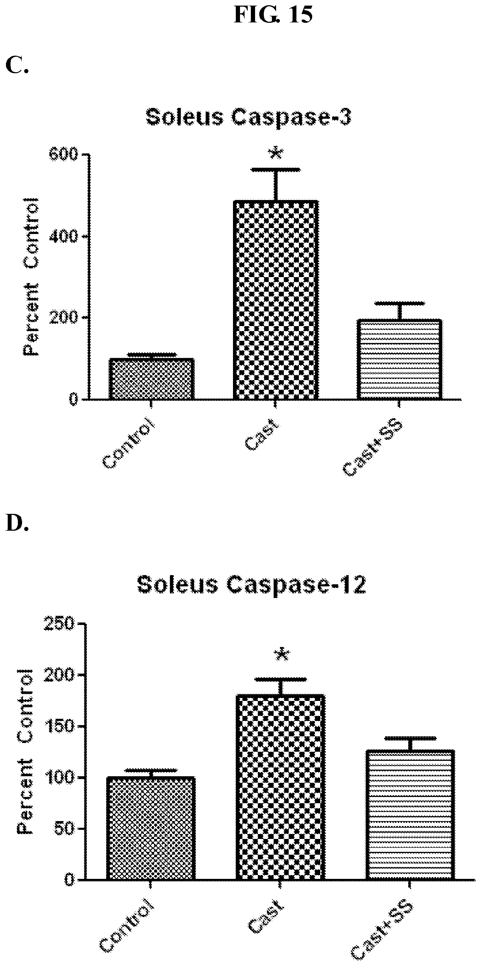

Casting also significantly increased oxidative damage in soleus muscle, as measured by lipid peroxidation *via* 4-hydroxynonenal (4-HNE). *See* **FIG. 15A****.** This effect was overcome by SS-31 administration. Moreover, casting significantly increased protease activity in the soleus muscle, which likely accounts for the muscle degradation and atrophy. As shown in **FIG. 15B-D,** calpain-1, caspase-3 and caspase-12 proteolytic degradation of muscle, respectively, were all prevented by SS-31.

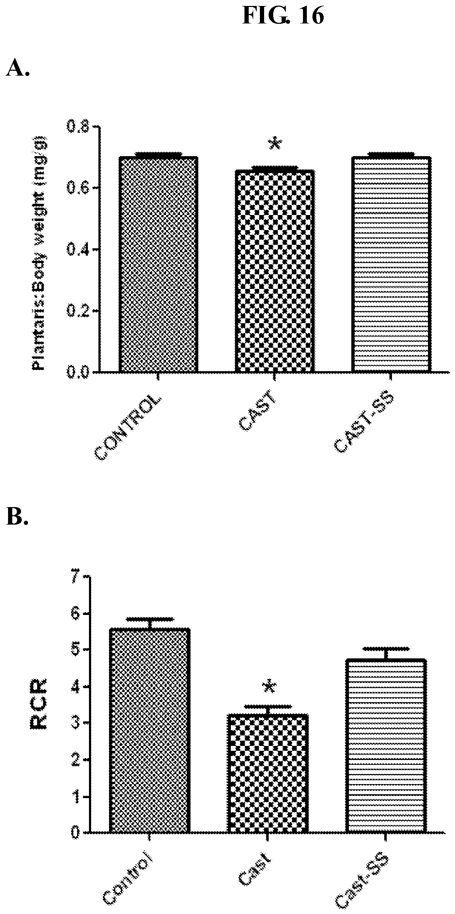

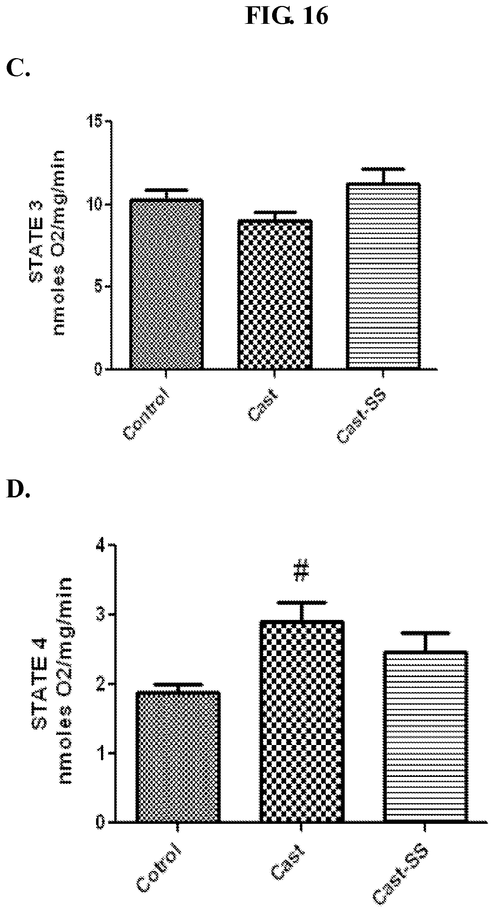

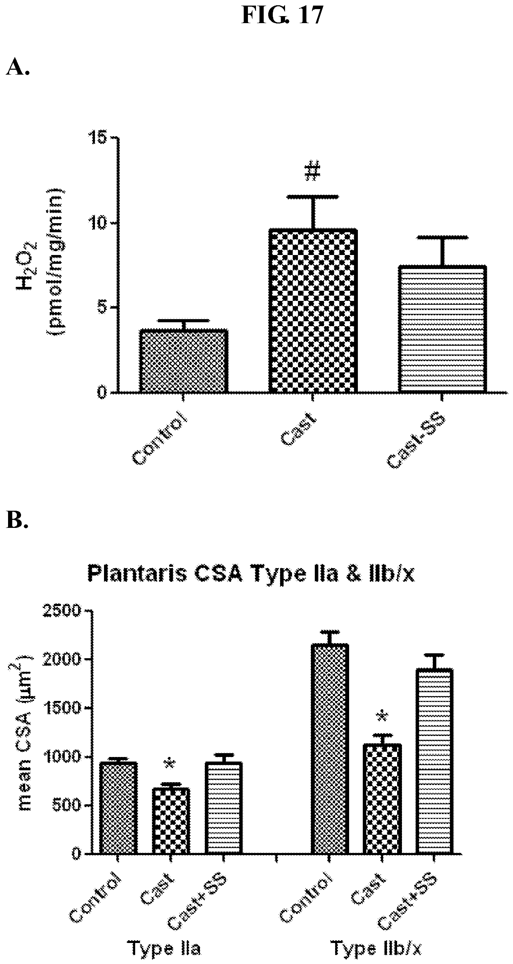

As illustrated by **FIG. 16A-D**, casting for 7 days leads to a significant decrease in plantaris muscle weight (**FIG**. **16A**), RCR **(****FIG. 16B****),** and mitochondrial state 4 respiration (**FIG**. **16D**), which is closely associated with ROS generation. All such effects were reversed *via* SS-31 administration. The casting did not have a significant effect on state 3 respiration. *See* **FIG. 16C****.** Similarly, casting for 7 days significantly increased H₂O₂ production by mitochondria isolated from plantaris muscle, which was prevented by SS-31. *See* **FIG. 17A-****B.** As shown in **FIG. 17B****,** SS-31 prevented cross sectional area loss for two types of fibers in the plantaris (type IIa and IIb/x).

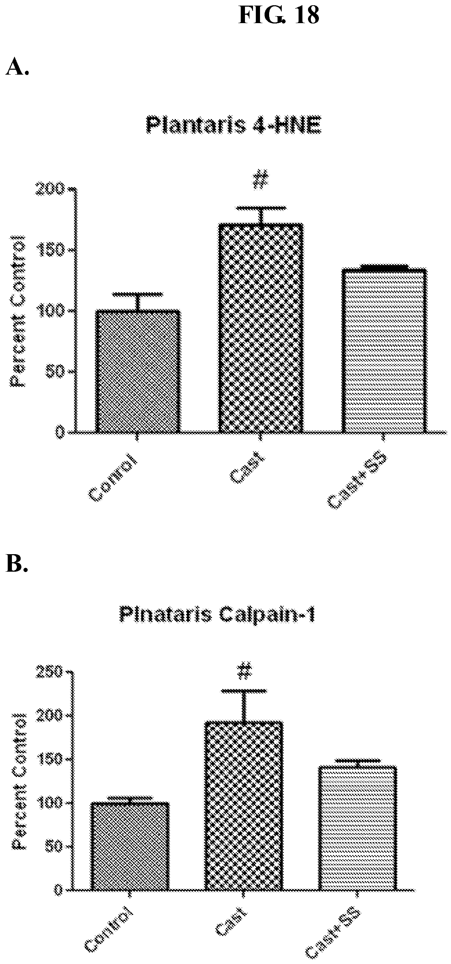

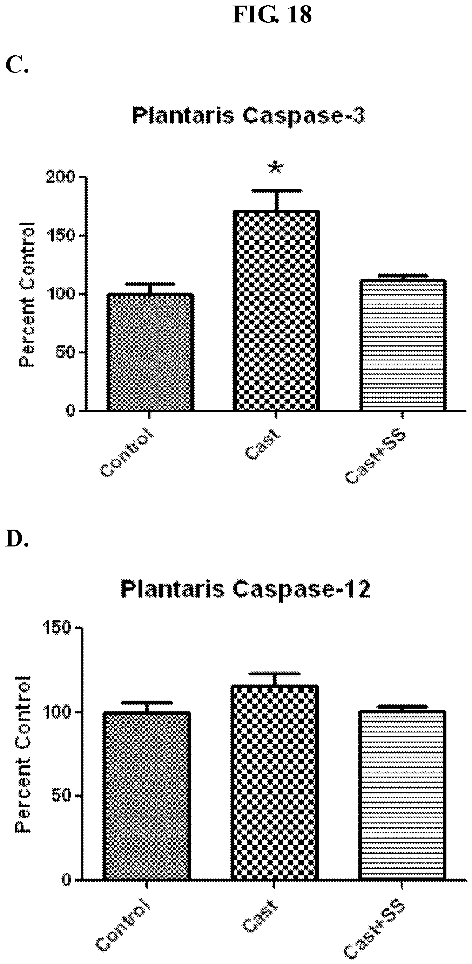

Casting also significantly increased oxidative damage in plantaris muscle, as measured by lipid peroxidation *via* 4-hydroxynonenal (4-HNE). *See* **FIG. 18A****.** This effect was overcome by SS-31 administration. Moreover, casting significantly increased protease activity in the soleus muscle, which likely accounts for the muscle degradation and atrophy. As shown in **FIG. 18B-D,** calpain-1, caspase-3 and caspase-12 proteolytic degradation of muscle were all prevented by SS-31, respectively.

In summary, results from these examples show that administering SS-31 to subjects with MV-induced or disuse-induced increases in mitochondrial ROS emissions not only reduces protease activity, but also attenuates skeletal muscle atrophy and contractile dysfunction. Treatment of animals with the mitochondrial-targeted antioxidant SS-31 was successful in preventing the atrophy in type I, IIa, and IIx/b fibers in the skeletal muscles described above. Further, prevention of MV-induced and disuses-induced increases in mitochondrial ROS emission also protected the diaphragm against MV-induced decreases in diaphragmatic specific force production at both sub-maximal and maximal stimulation frequencies. *See* **FIG. 3****.** Together, these results indicate that SS-31 can protect against and treat MV-induced and disuse-induced mitochondrial ROS emission in the diaphragm and other skeletal muscles.

The present invention is not to be limited in terms of the particular embodiments described in this application, which are intended as single illustrations of individual aspects of the invention. Many modifications and variations of this invention can be made without departing from its spirit and scope, as will be apparent to those skilled in the art. Functionally equivalent methods and apparatuses within the scope of the invention, in addition to those enumerated herein, will be apparent to those skilled in the art from the foregoing descriptions. Such modifications and variations are intended to fall within the scope of the appended claims. The present invention is to be limited only by the terms of the appended claims, along with the full scope of equivalents to which such claims are entitled. It is to be understood that this invention is not limited to particular methods, reagents, compounds compositions or biological systems, which can, of course, vary. It is also to be understood that the terminology used herein is for the purpose of describing particular embodiments only, and is not intended to be limiting.

In addition, where features or aspects of the disclosure are described in terms of Markush groups, those skilled in the art will recognize that the disclosure is also thereby described in terms of any individual member or subgroup of members of the Markush group.

As will be understood by one skilled in the art, for any and all purposes, particularly in terms of providing a written description, all ranges disclosed herein also encompass any and all possible subranges and combinations of subranges thereof. Any listed range can be easily recognized as sufficiently describing and enabling the same range being broken down into at least equal halves, thirds, quarters, fifths, tenths, *etc.* As a non-limiting example, each range discussed herein can be readily broken down into a lower third, middle third and upper third, *etc.* As will also be understood by one skilled in the art all language such as "up to," "at least," "greater than," "less than," and the like, include the number recited and refer to ranges which can be subsequently broken down into subranges as discussed above. Finally, as will be understood by one skilled in the art, a range includes each individual member. Thus, for example, a group having 1-3 peptides refers to groups having 1, 2, or 3 peptides Similarly, a group having 1-5 peptides refers to groups having 1, 2, 3, 4, or 5 peptides, and so forth.

All patents, patent applications, provisional applications, and publications referred to or cited herein are incorporated by reference in their entirety, including all figures and tables, to the extent they are not inconsistent with the explicit teachings of this specification.

Other embodiments are set forth within the following claims.

## Claims

1. A pharmaceutical composition for treating or preventing skeletal muscle infirmities in a mammalian subject, comprising a therapeutically effective amount of the peptide D-Arg-2',6'Dmt-Lys-Phe-NH₂ or a pharmaceutically acceptable salt thereof.

2. The composition of claim 1, wherein the skeletal muscle comprises diaphragmatic muscle.

3. The composition of claim 1, wherein the skeletal muscle infirmity results from mechanical ventilation (MV).

4. The composition of claim 3, wherein the duration of the MV is at least 10 hours.

5. The composition of claim 1, wherein the composition is formulated to be administered orally, topically, systemically, intravenously, subcutaneously, intraperitoneally, or intramuscularly.

6. A pharmaceutical composition for treating or preventing MV-induced diaphragm dysfunction in a mammalian subject, comprising a therapeutically effective amount of the peptide D-Arg-2',6'Dmt-Lys-Phe-NH₂ or a pharmaceutically acceptable salt thereof.

7. The composition of claim 6, wherein the MV is at least 10 hours.

8. The composition of claim 6, wherein the composition is formulated to be administered orally, topically, systemically, intravenously, subcutaneously, intraperitoneally, or intramuscularly.

9. A pharmaceutical composition for treating or preventing disuse-induced skeletal muscle atrophy in a mammalian subject, comprising a therapeutically effective amount of the peptide D-Arg-2',6'Dmt-Lys-Phe-NH₂ or a pharmaceutically acceptable salt thereof.

10. The composition of claim 9, wherein the skeletal muscle comprises soleus muscle or plantaris muscle, or both soleus and plantaris muscle.

11. The composition of claim 9, wherein the composition is formulated to be administered orally, topically, systemically, intravenously, subcutaneously, intraperitoneally, or intramuscularly.

12. A composition for treating a disease or condition **characterized by** increased oxidative damage in skeletal muscle of a mammalian subject in need thereof, the composition comprising:
an effective amount of D-Arg-2',6'Dmt-Lys-Phe-NH₂ or a pharmaceutically acceptable salt thereof, wherein the oxidative damage is associated with a variation in the gene expression or protein levels, activity, or degradation of one or more biomarkers selected from the group consisting of calpain, caspase-3, caspase 12, 20S proteasome, E3 ligases, atrogin-1/MAFbx, MuRF-1, αII-spectrin, sarcomeric protein, 4-HNE-conjugated cytosolic proteins, and protein carbonyls in myofibrillar proteins, compared to a control level.

13. The composition of claim 12, wherein the disease or condition **characterized by** increased oxidative damage comprises disuse-induced skeletal muscle atrophy or MV-induced diaphragm dysfunction.

14. The composition of claim 12, wherein the control level is the levels of the one or more biomarkers from a healthy individual not afflicted with disuse-induced skeletal muscle atrophy or MV-induced diaphragm dysfunction.

15. The composition of claim 12, wherein the composition is formulated to be administered orally, topically, systemically, intravenously, subcutaneously, intraperitoneally, or intramuscularly.
